# EUROPEAN PATENT APPLICATION

(11) **EP 4 417 219 A1**
(43) Date of publication of application: **21.08.2024**
(21) Application number: 23217985.3
(22) Date of filing: 19.12.2023
(51) Int. Cl.: A61K 41/00, A61P 35/00

(54) **MAGNETIC BIOMOLECULE-METAL ION SELF-ASSEMBLED COMPLEX FOR TREATMENT**

(30) Priority: 16.02.2023 KR 20230020922
(71) Applicant: Korea University Research and Business Foundation, Seoul 02841 (KR)
(72) Inventor: KANG, Heemin, 06344 Seoul (KR); JANG, Woo-Young, 04073 Seoul (KR)
(74) Representative: Meissner Bolte Partnerschaft mbB

(57) **Abstract**

A magnetic biomolecule-metal ion self-assembly complex for treatment includes an iron ion; and at least one ligand, wherein the ligand and the iron ion are reversibly self-assembled with each other or self-disassembled from each other, wherein the ligand and the iron ion are self-assembled with each other via a first bond to form a self-assembly, wherein the self-assembling is performed by at least one of the metal ion and the ligand, wherein the self-assembly includes a plural of self-assemblies, wherein the self-assemblies adjacent to each other self-bind to each other via a second bond to form the self-assembly complex. The magnetic biomolecule-metal ion self-assembly complex is effectively used for cancer treatment, osteoarthritis treatment, and bone defect treatment.

## Description

### BACKGROUND

### Field

The present disclosure relates to a magnetic biomolecule-metal ion self-assembly complex for treatment, and more specifically, to a magnetic biomolecule-metal ion self-assembly complex for treatment with improved treatment effect on cancer, osteoarthritis and bone defect.

### Description of Related Art

Recent anticancer treatments rely on chemically synthesized drugs. Chemotherapy drugs including doxorubicin which is typically used to treat cancer cause apoptosis of cancer cells to treat the cancer. On the contrary, each of doxorubicin, cisplatin, and methotrexate, which are representative drugs used in anticancer treatment is known to cause side effects on the heart, kidneys, and nerves. These side effects may cause symptoms depending on the patient's condition. This makes appropriate prescription thereof difficult. In particular, the doxorubicin which is widely used has a very narrow TI (therapeutic index) range as a concentration range in which the drug can be used. Thus, using a concentration thereof higher than this TI range causes fatal side effects.

Furthermore, these chemotherapy drugs may cause serious side effects, including hair loss, bone marrow suppression, vomiting, rash, stomatitis and hypersensitivity, allergies, heart damage, injection site tissue damage, radiation recall, and treatment-related leukemia. Furthermore, as cancer progresses, resistance to apoptosis increases, so that the treatment efficacy of the chemotherapy drugs may weaken.

Therefore, existing anticancer drugs have developed such that the drug targets the cancer and induces cancer cell death. Although many technologies have been developed, a variety of new methods are being studied which maintain their effectiveness in eliminating cancer cells, and causes fewer side effects and eliminate cancer that is resistant to cell death by the existing drugs.

Arthritis is one of the most common chronic diseases in the world. However, the pathophysiology of osteoarthritis has not been fully studied and thus there is currently no complete cure thereof. The osteoarthritis damages joint cartilage and causes extreme pain and disability in patients. Physiological regeneration of articular cartilage occurs only to a limited extent. Accordingly, various researchers are studying the pathophysiology of osteoarthritis using animal models and finding treatment methods thereof, and recently, methods of treating osteoarthritis using drugs have been implemented. The drugs used to treat osteoarthritis include hyaluronic acid, nonsteroidal anti-inflammatory drug (NSAID), and steroidal TAA (triamcinolone) to inhibit inflammation. However, there are almost no medications available in regenerating the cartilage.

Furthermore, various types of cells including mesenchymal stem cells (MSC), chondrocytes, macrophages, and fibroblasts exist in the bone j oint. The cytokine balance of the synovial fluid in the joint cavity is disrupted, causing pro-inflammatory cytokines to increase excessively compared to anti-inflammatory cytokines, thereby worsening the osteoarthritis.

Therefore, research is needed on drugs and treatment methods that are effective in treating the bone joint and can control the cytokine balance within the bone joint to a level of the normal bone joint.

It is very difficult to treat an extensive bone defect in ilium fracture including the femur clinically. Recently, the induced membrane technique is used as a treatment method for such a wide range of the bone defect. In the induced membrane method, all dead or infected bones are removed through primary surgery, and then the removed area is filled with bone cement such as calcium phosphate, and the patient waits for about 4 to 6 weeks for the formation of the induced membrane that is generated with reaction to the bone cement, and then a secondary surgery is performed to remove the bone cement, and a large amount of an autologous bone is transplanted into the removed area. The price of BMP used in this induced membrane method is very high to be used in actual treatment, and there are clinical problems related to the induced membrane method that should be overcome. Furthermore, the induced membrane method is performed in a limited manner using the autologous bone transplantation including BMP. There may be insufficient autogenous bone which can be obtained from the patient. When the bone defect site is too extensive, an amount of all of the autologous bones including the pelvic bone and tibia that can be harvested is insufficient.

Such autologous bone transplantation causes many complications such as delayed rehabilitation due to pain at the harvest site, bleeding, infection, and iatrogenic fractures. Thus, research on graft materials that may replace the autologous bone in the induced membrane method is necessary.

### SUMMARY

A purpose of the present disclosure is to provide a magnetic biomolecule-metal ion self-assembly complex for treatment which can be self-assembled in various forms based on the type of the substance in the living body, and does not case side reactions in the living body.

Furthermore, another purpose of the present disclosure is to provides a magnetic biomolecule-metal ion self-assembly complex for treatment which is not toxic to living organisms, can be self-assembled into various forms depending on the type of the ligand, and in which a position of the magnetic biomolecule-metal ion self-assembly complex located in the body may be easily controlled using means out of the body to effectively treat the target location.

Purposes according to the present disclosure are not limited to the above-mentioned purpose. Other purposes and advantages according to the present disclosure that are not mentioned may be understood based on following descriptions, and may be more clearly understood based on embodiments according to the present disclosure. Further, it will be easily understood that the purposes and advantages according to the present disclosure may be realized using means shown in the claims or combinations thereof.

According to one aspect of the present disclosure, embodiments of the present disclosure relate to a magnetic biomolecule-metal ion self-assembly complex for treatment.

According to the present disclosure as discussed above, the magnetic biomolecule-metal ion self-assembly complex may be realized which can perform target treatment targeting a local site, has no side effects, and easily induces the cell death and thus is effective in the cancer treatment.

Furthermore, according to the present disclosure, the magnetic biomolecule-metal ion self-assembly complex for treatment may be realized that can regulate the cytokine balance within the bone joint having the osteoarthritis to the level of the normal bone joint in order to prevent the osteoarthritis from worsening.

Furthermore, according to the present disclosure, the magnetic biomolecule-metal ion self-assembly complex for treatment which effectively treats bone defects, and has no side effects may be realized.

Effects of the present disclosure are not limited to the effects mentioned above, and other effects not mentioned will be clearly understood by those skilled in the art from the descriptions below.

### BRIEF DESCRIPTION OF DRAWINGS

FIG. 1 is a diagram schematically showing a process in which an iron ion and ATP are self-assembled with each other into a self-assembly according to one embodiment of the present disclosure.
FIG. 2 is a diagram schematically showing a process in which an iron ion and AMP are self-assembled with each other into a self-assembly according to one embodiment of the present disclosure.
FIG. 3 shows a SEM image of the Fe-ATP self-assembly and a result of measuring paramagnetism thereof.
FIG. 4 is an SEM image of the Fe-ATP self-assembly with each of average diameters of 150 nm and 70 nm according to one embodiment of the present disclosure.
FIG. 5 is a TEM image of the Fe-AMP self-assembly.
FIG. 6 is a diagram schematically showing the induction of ferroptosis in cancer cells using the Fe-ATP self-assembly.
FIG. 7 is a graph showing the iron ion release of the Fe-ATP self-assembly at pH 5.5 or pH 7.4 measured using an iron assay kit.
FIG. 8 shows an intracellular ROS concentration as measured via DCFDA staining according to each of conditions.
FIG. 9 is a result of analyzing a IVIS L-012 ROS signal.
FIG. 10 shows a result of identifying ferroptosis of cancer cells via continuous Fe²⁺ release from a magnetically targeted Fe-ATP self-assembly.
FIG. 11 shows that the magnetically targeted Fe-ATP self-assembly effectively treats osteosarcoma cancer, as doxorubicin does.
FIG. 12 shows a result of identifying toxicity after transplanting the Fe-ATP self-assembly into the body.
FIG. 13 shows a result of identifying the chondroprotective effect of the magnetically targeted Fe-ATP self-assembly.
FIG. 14 is a graph showing a cumulative ATP release profile from the Fe-ATP self-assembly as measured by HPLC.
FIG. 15 shows a result of identifying polarization of macrophages via disassembling of the Fe-ATP self-assembly using immunofluorescence images.
FIG. 16 shows a result of identifying the polarization of macrophages via disassembling of the Fe-ATP self-assembly using flow cytometry.
FIG. 17 shows a result of identifying the disassembling of the Fe-ATP self-assembly by western blotting.
FIG. 18 shows C-Arm and 3-D micro CT images of the knee bone joint to identify the effect of osteoarthritis treatment by the Fe-ATP self-assembly.
FIG. 19 shows a 2-D micro-CT image, and H&E staining and safranin-o (S-O) staining images of the knee joint of an osteoarthritis model at 8 weeks.
FIG. 20 shows H&E staining, safranin-o (S-O) staining, and collagen X staining images of an osteoarthritis model at week 8.
FIG. 21 shows iNOS and Arg-1 staining images of a synovial membrane of the knee joint of an osteoarthritis model at 8 weeks.
FIG. 22 shows a toxicity result of the Fe-ATP self-assembly identified in the osteoarthritis rat model (rat model).
FIG. 23 is a diagram schematically showing a bone regeneration effect of a 3D-shaped Fe-AMP self-assembly.
FIG. 24 is an SEM image showing a micro pore structure in which 3D-shaped Fe-AMP self-assemblies are aggregated with each other.
FIG. 25 is a diagram showing magnetically targeting of a 3D-shaped Fe-AMP self-assembly.
FIG. 26 is a graph showing AMP released over time from the Fe-AMP self-assembly.
FIG. 27 shows a result of identifying, based on immunofluorescence images, that osteogenic differentiation is promoted by adenosine obtained via decomposition of the AMP released from the 3D-shaped Fe-AMP self-assembly.
FIG. 28 shows the Western blotting results identifying osteogenic differentiation by CD73-mediated adenosine obtained via decomposition of the AMP released from the 3D-shaped Fe-AMP self-assembly.
FIG. 29 shows a C-Arm image of the femur to identify fractures at 0, 2, 4, 6, and 8 weeks.
FIG. 30 shows a micro-CT image of the femur to identify fractures at 8 weeks.
FIG. 31 shows H&E, osteocalcin, and TRAP staining images of a fracture.
FIG. 32 shows a result of identifying the toxicity of the 3D-shaped Fe-AMP self-assembly in vivo.

### DETAILED DESCRIPTIONS

The specific details of other embodiments are contained in the detailed description and drawings.

Advantages and features of the present disclosure, and a method to achieve the same will become apparent with reference to the embodiments described below in detail in conjunction with the accompanying drawings. However, the present disclosure is not limited to the embodiments disclosed below and may be implemented in a variety of different forms. Unless otherwise specified in following descriptions, all of numbers, values, and/or expressions indicating ingredients, reaction conditions, and contents of ingredients in the present disclosure are, in essence, approximations thereof based on various uncertainties in measurements which occur in obtaining the numbers, values, and/or expressions. Thus, the numbers, values and/or expressions should be understood as being modified by a term "about" in all instances. Further, where a numerical range is disclosed in the present description, the range is continuous and includes a minimum value and a maximum value of the range, unless otherwise indicated. Further, where the number or the value refers to an integer, the range includes all of integers included between the minimum and the maximum of the range, unless otherwise indicated.

Further, in the present disclosure, when a variable is contained in a range, the variable will be understood to include all values within a stated range including stated endpoints of the range. For example, a range of "5 to 10" includes values of 5, 6, 7, 8, 9, and 10, as well as any subranges such as 6 to 10, 7 to 10, 6 to 9, 7 to 9, etc. It will be understood that the variable includes any value between valid integers in a stated range such as 5.5, 6.5, 7.5, 5.5 to 8.5, and 6.5 to 9, etc. For example, a range "10% to 30%" includes all of integer values such as 10%, 11%, 12%, 13%, 30%, etc. as well as any subranges such as 10% to 15%, 12% to 18%, or 20% to 30%, etc. It will be understood that the range includes any value between valid integers within the stated range such as 10.5%, 15.5%, 25.5%, etc.

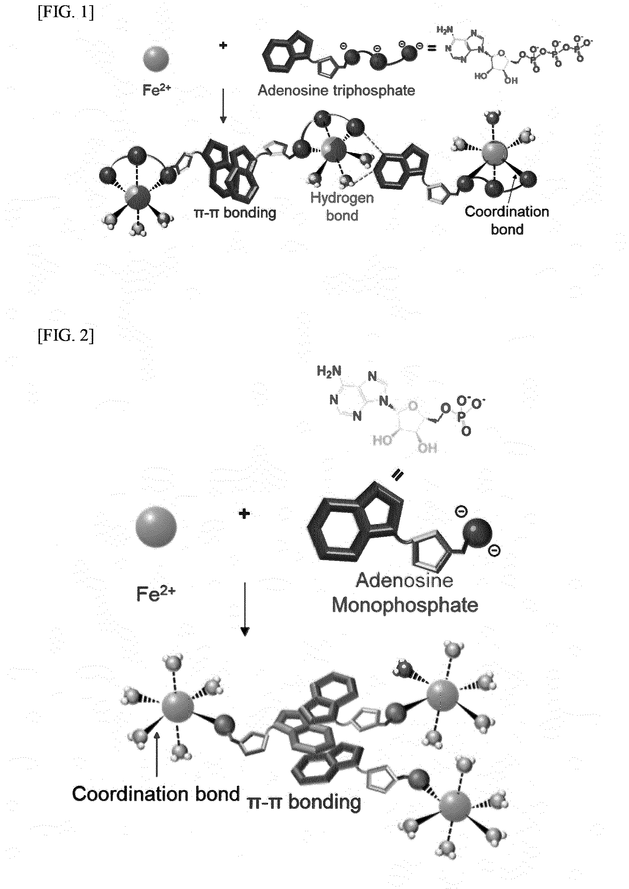

FIG. 1 is a diagram schematically showing a process in which an iron ion and ATP are self-assembled with each other into a self-assembly according to one embodiment of the present disclosure. FIG. 2 is a diagram schematically showing a process in which an iron ion and AMP are self-assembled with each other into a self-assembly according to one embodiment of the present disclosure.

Referring to FIG. 1 and FIG. 2, a magnetic biomolecule-metal ion self-assembly complex for treatment according to the present embodiment may include an iron ion; and at least one ligand, wherein the ligand and the iron ion are reversibly self-assembled with each other or self-disassembled from each other, wherein the ligand and the iron ion are self-assembled with each other via a first bond to form a self-assembly. Furthermore, in the magnetic biomolecule-metal ion self-assembly complex for the treatment, the self-assembling for formation of the self-assembly may be achieved by at least one of the metal ion and the ligand, wherein the complex includes a plurality of self-assemblies, wherein neighboring self-assemblies may be self-bind to each other via a second bond to form the complex.

In the present embodiment, the magnetic biomolecule-metal ion self-assembly complex for treatment (hereinafter, a biomolecule-metal ion self-assembly complex) is effective in treating diseases, etc., has magnetism, and includes the self-assembly in which the biomolecule and the metal ion are self-assembled with each other. At least two self-assemblies may bind to each other form the complex.

The ligand may include at least one of phosphate and phosphonate. Specifically, the ligand may include at least one of AMP, ADP, ATP, TMP, TDP, TTP, CMP, CDP, CTP, GMP, GDP, GTP, UMP, UDP, UTP, DNA, RNA, AEP (2-aminoethylphosphonic acid), threose nucleic acid (TNA), glycol nucleic acid (GNA), 1,5-anhydrohexitol nucleic acid (HNA), 1,5-anhydroatritol nucleic acid (ANA), 2'-deoxy-2'-fluoroarabino nucleic acid (FANA), and CeNA (cyclohexenyl nucleic acid). More specifically, the ligand may include at least one of AMP (adenosine monophosphate) and ATP (adenosine triphosphate).

The ligand and the iron ion self-bind to each other via the first bond to form the self-assembly. The neighboring self-assemblies may self-bind to each other via the second bond to form the biomolecule-metal ion self-assembly complex. In the biomolecule-metal ion self-assembly complex, the first bond may include a coordination bond, and the second bond may include at least one of a hydrogen bond and π-π interaction.

In FIG. 1, the iron ion (Fe²⁺) may be self-assembled with ATP as the ligand via a coordination bond to form the Fe-ATP self-assembly. The Fe-ATP self-assemblies may be self-assembled with each other via at least one of the hydrogen bond and the π-π interactions between neighboring Fe-ATP self-assemblies to form the magnetic biomolecule-metal ion self-assembly complex for treatment according to one embodiment of the present disclosure.

Furthermore, in FIG. 2, the iron ion (Fe²⁺) may be self-assembled with AMP as the ligand via a coordination bond to form the Fe-AMP self-assembly. The Fe-AMP self-assemblies may be self-assembled with each other via at least one of the hydrogen bond and the π-π interactions between neighboring Fe-AMP self-assemblies to form the magnetic biomolecule-metal ion self-assembly complex for treatment according to one embodiment of the present disclosure.

The iron ion may coordination-bond with the phosphate site of ATP or AMP and water (H₂O) to form a metal complex. Furthermore, the aromatic ring of the adenine site of ATP or AMP may form a π-π interaction. Furthermore, the water molecule binding to the iron ion and the nitrogen of the adenine site may form a hydrogen bond. The biomolecule and the metal ion may be self-assembled with each other using at least one of coordination bonds, π-π interactions, and hydrogen bonds to form the complex including one or more self-assembly.

The biomolecule and the metal ion or the neighboring self-assemblies may be self-assembled with each other during a first time or may be self-disassembled from each other for a second time under a physiologically relevant condition. The first time may be in a range from 1 minute to 24 hours, and the second time may be in a range from 1 day to 90 days.

The physiologically relevant condition refers to temperature, pH, osmotic pressure, ionic strength, viscosity, and similar biochemical parameters that are compatible with a viable organism and/or are normally present within a viable culturing yeast cell or a mammalian cell. For example, in the case of human cells, the physiologically relevant condition may include a temperature of approximately 37°C, for example, a temperature of 35 to 39°C, a range of 1kPa to 200kPa, and a pH of about 7. Specifically, the physiologically relevant condition means a condition that is not toxic to living organisms or animals, and a condition under which living organisms or animals may be maintained.

The biomolecule and the metal ion or the neighboring self-assemblies may be self-assembled with each other during the first time, that is, for 1 minute to 24 hours. When the first time is smaller than 1 minute, there is a problem because the biomolecule and the metal ion or the neighboring self-assemblies may not be stably self-assembled with each other via the first bond or the second bond. The biomolecule and the metal ion or the neighboring self-assemblies may be self-assembled with each other for a time duration within 24 hours in a sufficient manner. The time exceeding 24 hours may reduce efficiency. Specifically, the first time may be in a range of about 1 minute to 20 hours, or about 1 minute to 15 hours, or about 1 minute to 10 hours, or about 1 minute to 5 hours, or about 1 minute to 2 hours, or about 1 minute to 30 minutes.

The biomolecule and the metal ion or the neighboring self-assemblies may be self-disassembled from each other for the second time, that is, 1 day to 90 days. As the biomolecule and the metal ion or the neighboring self-assemblies are self-disassembled from each other for the second time, the iron ions or ligands may be released to stably produce a therapeutic effect. Specifically, the second time may be in a range of about 1 day to 45 days, or about 1 day to 30 days, or about 1 day to 15 days, or about 1 day to 10 days.

When the ligand is ATP (adenosine triphosphate), the magnetic biomolecule-metal ion self-assembly complex for the treatment may be provided in an individual spherical shape. Furthermore, when the ligand is AMP (adenosine monophosphate), the magnetic biomolecule-metal ion self-assembly complex for the treatment may be provided in a form of a three-dimensional aggregate with micro pores in which the plurality of self-assemblies are aggregated with each other.

Specifically, when the ligand is ATP, the biomolecule-metal ion self-assembly complex has a spherical shape and may be formed so as to have a uniform diameter. The diameter of the biomolecule-metal ion self-assembly complex may vary depending on at least one of the ligand concentration and the iron ion concentration. Specifically, when the concentration of the iron ion is high, the diameter of the self-assembly may increase. Furthermore, when the concentration of the ligand is high, the diameter of the self-assembly may be increased. When the biomolecule-metal ion self-assembly complex according to the present embodiment uses ATP as the ligand, the diameter of the biomolecule-metal ion self-assembly complex may be controlled by controlling the concentration of iron ions and/or the concentration of ATP.

When the ligand is AMP, the biomolecule-metal ion self-assembly complex may be provided in the form of the three-dimensional aggregate. Specifically, the biomolecule-metal ion self-assembly complex may be provided in a form having a volume with a plurality of micro pores therein. For example, even when the biomolecule-metal ion self-assembly complex uses AMP as the ligand, the size of the three-dimensional aggregate may be controlled by controlling the concentration of the iron ion and/or the concentration of AMP. Specifically, when increasing the concentration of iron ion or increasing the concentration of AMP, the size of the three-dimensional aggregate may be increased. The size of the three-dimensional aggregate may be in a range of 100 nm to 100 cm. Specifically, the size of the three-dimensional aggregate may refer to the average diameter when the aggregate has a sphere shape. The size of the three-dimensional aggregate may refer to an average value of the average diameter of the cross section and the height when the aggregate has a cylindrical shape. The size of the three-dimensional aggregate may refer to an average value of a length of a side and a height when the aggregate has a polygon shape. The size of the three-dimensional aggregate may be in a range of about 100nm to 80cm, or about 100nm to 50cm, or about 100nm to 30cm, or about 100µm to 30cm, or about 200µm to 30cm, about 300µm to 30cm, or about 400µm to 30cm, or 500µm to 30cm, or 500µm to 20cm, or 500µm to 10cm.

The self-assembly has paramagnetic properties, and movement of the self-assembly may be controlled by applying an external magnetic field thereto. Specifically, the biomolecule-metal ion self-assembly complex may be present in vivo or in vitro, and the movement of the self-assembly constituting the biomolecule-metal ion self-assembly complex may be controlled by applying an external magnetic field thereto. Accordingly, the position of the biomolecule-metal ion self-assembly complex according to the present embodiment may be controlled to a target location. Specifically, after injection the biomolecule-metal ion self-assembly complex into the body, the biomolecule-metal ion self-assembly complex may be displaced to the target cell or tissue to directly exert a treatment effect thereon.

The self-disassembling of the self-assembly may be promoted under at least one of a condition containing a chelating agent, a strong acid condition, and a strong base condition.

In the condition containing the chelating agent, the chelating agent may be at least one of ethylenediaminetetraacetic acid (EDTA), bipyridyl, and ferrozine.

The chelating agent may form a bond with the iron ion instead of the ligand to promote the self-disassembling of the self-assembly. The chelating agent may be a substance that has a higher affinity to the iron ion than to the ligand, and may be, for example, EDTA.

The pH of the strong acid condition may be in a range of 2 to 5, and the pH of the strong base condition may be in a range of 9 to 12. Specifically, the pH of the strong acid condition may be in a range of 3 to 4, and the pH of the strong base condition may be in a range of 10 to 11. For example, the strong acid may be hydrochloric acid (HCl), and the strong base may be NaOH.

The strong acid or strong base condition may promote disassembling of the assembly between the iron ion and the ligand, and may also promote self-disassembling of the self-assembly complex.

The self-assembly may have paramagnetic properties. As a volume of the self-assembly or the biomolecule-metal ion self-assembly complex increases, the magnetic moment may increase. Therefore, as the self-assembly complex is larger, the attractive force (or repulsive force) caused by the magnetic field applied thereto may be greater. Specifically, when the magnetic field is applied to the biomolecule-metal ion self-assembly complex in vivo and in vitro, the movement of the biomolecule-metal ion self-assembly complex may be controlled by the magnetic field. As the size of the biomolecule-metal ion self-assembly complex increases, the movement speed of the biomolecule-metal ion self-assembly complex may be further increased. Furthermore, the biomolecule-metal ion self-assembly complex according to the present embodiment is magnetic only when the magnetic field is applied to the biomolecule-metal ion self-assembly complex. Thus, the biomolecule-metal ion self-assembly complex may be easily controlled in vivo and in vitro.

The biomolecule-metal ion self-assembly complex according to the present embodiment may induce ferroptosis and kill specific cancer cells.

Typically, anticancer treatment relies on chemically synthesized drugs. The representative drugs of the chemically synthesized drugs such as doxorubicin, cisplatin, and methotrexate cause side effects on the heart, kidneys, and nerves. Furthermore, in order to reduce damage to normal tissue in the process of treating cancer, immune treatment agents and target treatment agents that target cancer have been developed and used. The patients having sarcoma have different target substances. Thus, it is difficult to use a target treatment agent thereon. The immunotherapy agent is very expensive, thus making it very difficult to use the same in actual treatment.

On the contrary, the biomolecule-metal ion self-assembly complex according to the present embodiment is not toxic to living organisms and may target and move the cancer cells under externally applied magnetism thereto. Furthermore, the biomolecule-metal ion self-assembly complex may induce ferroptosis in cancer cells to effectively kill cancer cells, have no effect on surrounding normal cells, and may be used stably without toxicity in the body. The ferroptosis may induce cell death in a different scheme from the cell death induced by oxidative damage to cell phospholipids.

In the biomolecule-metal ion self-assembly complex according to the present embodiment, the self-assembly may generate an excess amount of reactive oxygen species (ROS) under a condition containing hydrogen peroxide at a concentration of 10 µM or higher. The amount of the produced reactive oxygen species may be proportional to the self-assembly concentration and the concentration of hydrogen peroxide. The amount of reactive oxygen species as produced when the self-assembly and hydrogen peroxide are injected to cancer cells may be larger than or equal to 4 times of that before the self-assembly and hydrogen peroxide are injected to the cancer cells. Thus, the reactive oxygen species may effectively induce death of cancer cells via the ferroptosis. Specifically, the concentration of hydrogen peroxide may be approximately in a range of 10µM to 10M, or 10µM to 5M, or 20µM to 10M, or 30µM to 10M, or 40µM to 10M, or 50µM to 10M.

Generally, the concentration of hydrogen peroxide in normal cells is approximately 20 nM or smaller, and the concentration of hydrogen peroxide in cancer cells is approximately 10 µM or greater, for example, in a range of 10 µM to 100 µM, which is higher than that of the normal cells. Therefore, the biomolecule-metal ion self-assembly complex according to this example self-disassembles only in cancer cells having high hydrogen peroxide concentration to release Fe²⁺. The released Fe²⁺ may generate excessive ROS based on a Fenton reaction. The generated excessive ROS may lead to cancer cell death via the ferroptosis.

The reactive oxygen species oxidizes cellular phospholipids and inhibits GPX4 (glutathione peroxidase 4) or System xc-cystine/glutamate antiporter (Xc) to induce death of cancer cells via the ferroptosis. The GPX4 is an enzyme that is present in the cell, and repairs oxidized phospholipids. GPX4 is regulated by System xc - cystine/glutamate antiporter (Xc).

The biomolecule-metal ion self-assembly complex according to the present embodiment may include the iron ion. The iron ion may be released via self-disassembling of the self-assembly that constitutes the biomolecule-metal ion self-assembly complex. The iron ion is a biological material that reacts with hydrogen peroxide at the high concentration in cancer cells to generate the ROS via the Fenton reaction. Then, the ROS may damage the cell phospholipids via an oxidation reaction, thereby causing ferroptosis of cancer cells. Specifically, the ROS oxidizes cellular phospholipids, and thus, an excessive amount of oxidized phospholipids is generated, leading to decrease in GPX4 and Xc. When the oxidized phospholipids are excessively generated and thus may not be repaired by GPX4, the ferroptosis of cells is induced.

When ferroptosis of the above cells acts on organs other than cancer cells, cardiac side effects (cardiomyopathy, etc.) may occur. Therefore, in the process of treating cancer by inducing ferroptosis using artificially synthesized drugs such as doxorubicin, side effects even occur in normal cells surrounding the cancer cells.

On the contrary, the biomolecule-metal ion self-assembly complex according to the present embodiment may target a local site under an externally applied magnetic field thereto, thereby selectively removing only cancer cells. The biomolecule-metal ion self-assembly complex is composed of the biomolecules and iron ions that are not toxic to the body, and thus is not toxic to the body, has no side effects, and may be used continuously for a long period of time. Furthermore, the biomolecule-metal ion self-assembly complex may induce the ferroptosis specifically to the cancer cells without affecting normal cells to kill the cancer cells, thereby effectively treating the cancer.

In the biomolecule-metal ion self-assembly complex, the self-disassembly rate of the self-assembly may increase under an acidic condition. Therefore, the self-disassembly rate of the self-assembly may be low in normal cells having a neutral condition, while the disassembly rate thereof may be high in cancer cells having the acidic condition. Furthermore, cancer cells may have a relatively higher hydrogen peroxide concentration than that of the normal cells, and the acidic condition. Thus, the biomolecule-metal ion self-assembly complex nay generate ROS specifically to the cancer cells. That is, in the biomolecule-metal ion self-assembly complex according to the present embodiment, the disassembly rate of the self-assembly in the cancer cells may be much higher than that in the normal cells. Thus, most of the iron ions released via the self-disassembling of the self-assembly, and thus the Fenton reaction causing the ROS reaction act on the cancer cells and therefore has little effect on the normal cells.

Furthermore, the biomolecule-metal ion self-assembly complex according to the present embodiment enables local site targeting, has fewer side effects, and exhibits comparable effectiveness in killing cancer cells, and has no cardiac side effects, compared to doxorubicin as a representative conventional drug for cancer treatment.

In the biomolecule-metal ion self-assembly complex according to the present embodiment, the self-assembly has paramagnetic properties, and thus, its movement is controlled by applying an external magnetic field thereto. Thus, under application of the external magnetic field thereto, the self-assembly may move to target cancer cells and induce death of cancer cells via the ferroptosis.

When the self-assembly induces death of cancer cells via the ferroptosis, the self-assembly may kill cancer cells at a killing ability similar to that of doxorubicin. Furthermore, while the self-assembly induces death of cancer cells via the ferroptosis, the self-assembly may not be toxic to normal cells.

In the biomolecule-metal ion self-assembly complex according to the present embodiment, the self-assembly is used for cancer treatment. The cancer may include at least one selected from a group consisting of breast cancer, colon cancer, rectal cancer, lung cancer, colon cancer, thyroid cancer, oral cancer, pharynx cancer, larynx cancer, cervical cancer, brain cancer, ovarian cancer, bladder cancer, kidney cancer, liver cancer, pancreatic cancer, prostate cancer, skin cancer, tongue cancer, uterine cancer, stomach cancer, bone cancer, and blood cancer.

In the biomolecule-metal ion self-assembly complex according to another embodiment of the present disclosure, the self-assembly may be formed via self-assembling of the iron ion and the ligand via the first bond. The self-assemblies may be self-assembled with each other via the second bond between neighboring self-assemblies. In this regard, the ligand may include ATP (adenosine triphosphate). When the self-assembly is disassembled, the ATP may be released to promote M2 polarization of macrophages via the P2Y1 receptor. Furthermore, the self-assembly promotes M2 polarization of macrophages distributed in the synovial membrane of bone joints. The M2 polarization of the macrophages may have anti-inflammatory activity of the synovial fluid of bone joints.

Typically, arthritis treatment is performed so as to prevent inflammation. In this regard, there are almost no drugs that regenerate the cartilage. Various types of cells including MSCs, chondrocytes, macrophages, and fibroblasts exist in the bone joints. When the cytokine balance of the synovial fluid in the joint cavity is disrupted, pro-inflammatory cytokines that promote inflammation increases excessively compared to anti-inflammatory cytokines, such that the osteoarthritis gets worse.

The self-assembly may include ATP as a biological substance as the ligand. The ATP may bond with various cell membrane receptors to control cell functions. The type of cell membrane receptor that bonds with ATP may be controlled depending on the concentration of ATP. The macrophages may greatly affect the balance of cytokines in synovial fluid within bone joints. The synovial fluid within the bone joint where osteoarthritis occurs contains approximately 0.5 nM of ATP. When the ATP concentration is slightly increased (e.g., so as to be several µM) using the Fe-ATP self-assembly, M2 polarization may be promoted via the P2Y receptor series, thereby promoting the secretion of anti-inflammatory cytokines. However, when the ATP concentration is very high (e.g., several mM), this promotes M1 polarization via the P2X receptor series to promote the secretion of pro-inflammatory cytokines.

Specifically, when the biomolecule-metal ion self-assembly complex according to the present embodiment is injected into the site where osteoarthritis occurs, the self-assembly is disassembled to release ATP. The ATP is controlled so as to have a concentration of 5 to 10µM, thereby promoting M2 polarization of macrophages distributed in the synovial membrane of bone joints. The M2 polarization of the macrophages may result in anti-inflammatory activity of the synovial fluid of bone joints. Furthermore, when ATP released via the self-disassembling of the self-assembly controls the M2 polarization of the macrophages, the M2 polarization of the macrophages may not be toxic to normal cells while having the anti-inflammatory activity of the synovial fluid of bone joints. Furthermore, the self-assembly may prevent, improve, or treat osteoarthritis by maintaining an anti-inflammatory environment in the synovial fluid of bone joints to protect the bone cartilage.

The self-assembly has paramagnetic properties, and thus, its movement may be controlled by applying an external magnetic field thereto. Therefore, after injecting the biomolecule-metal ion self-assembly complex into the body, the external magnetic field may be applied thereto such that the self-assembly moves to the target bone joint site to protect the bone cartilage to prevent, improve, or treat osteoarthritis.

The biomolecule-metal ion self-assembly complex according to another embodiment of the present disclosure may include at least one self-assembly, wherein the iron ion and the ligand may be self-assembled with each other via the first bond. In this regard, the ligand may include AMP (adenosine monophosphate). The biomolecule-metal ion self-assembly complex may be formed by aggregating a plurality of self-assemblies with each other into a three-dimensional aggregate with micro pores. The three-dimensional aggregate may have an average diameter of 500µm to 10cm. The self-assembly complex may be transplanted to the bone defect site in the form of the three-dimensional aggregate and thus may be used as a bone graft material to restore the defective bone tissue. For example, when the average diameter of the biomolecule-metal ion self-assembly complex formed in the three-dimensional aggregate is smaller than 500 µm, it is too small to be used as the bone graft material. When the average diameter of the biomolecule-metal ion self-assembly complex formed in the three-dimensional aggregate exceeds 10cm, the strength of the three-dimensional aggregate is reduced.

A shape of the three-dimensional aggregate may be of various three-dimensional structures depending on a shape of a used mold, and may include a plurality of micro pores therein. For example, the three-dimensional aggregate may have a mesh structure in which the micro pores are three-dimensionally interconnected to each other.

When bone defects occur in a wide area in a fracture of ilium including the femur, it is very difficult to clinically treat the same. Currently, various bone substitutes such as allogeneic bone, xenogeneic bone, and synthetic bone are used along with autologous bone transplantation. However, the higher the proportion of bone substitutes, the higher the bone resorption percentage, which lowers the treatment effect. Furthermore, the autologous bone transplantation has the problem of causing many complications such as delayed rehabilitation due to pain at the harvest site, bleeding, infection, and iatrogenic fractures.

In the biomolecule-metal ion self-assembly complex according to the embodiment of the present disclosure, the self-assembly may be self-disassembled into the iron ion and the AMP and thus the AMP may be released therefrom. The released AMP promotes osteogenic differentiation of mesenchymal stem cells (MSCs) via signaling of the adenosine receptor of the mesenchymal stem cells (MSCs). The osteogenic differentiation of mesenchymal stem cells (MSCs) may promote bone generation.

When the biomolecule-metal ion self-assembly complex is implanted into a bone defect site, the complex may act as a graft material for the bone defect site and simultaneously release AMP via the self-disassembling of the self-assembly. The AMP released from the self-assembly may fill the bone defect site and promote MSC attachment and osteogenic differentiation of MSC.

Furthermore, the self-assembly according to the present embodiment has paramagnetic properties, and its movement may be controlled by applying an external magnetic field thereto. When the self-assembly is implanted into a bone defect site in a form of the three-dimensional aggregate, the self-assembly may move in at least one of one direction and the other direction under application of the external magnetic field thereto.

The self-assembly may be transplanted to the bone defect site, and host cells may attach to the surface of the self-assembly to form a skeleton. Furthermore, the self-assembly has paramagnetic properties, and its movement may be controlled by applying an external magnetic field thereto. For example, when the self-assembly is implanted into a bone binding site, the self-assembly may move under the application of the external magnetic field thereto. At this time, the host cells attached to the surface of the self-assembly may move together therewith.

The self-assembly may be transplanted to the bone defect site, and the self-assembly may be self-disassembled into the iron ion and the AMP such that the AMP may be released therefrom for 1 to 70 days. The AMP released from the self-assembly may decompose into adenosine on the surface of the host cell to promote the signaling of the adenosine receptor on the surface of the host cell. For example, the adenosine receptor may include the adenosine A2B receptor.

When the self-assembly is transplanted to a bone defect site to regenerate a defective bone tissue, the assembly may promote the bone regeneration at a similar level to that of the calcium-phosphate cement as a bone defect graft material. Furthermore, the self-assembly may not be toxic to normal cells while it is transplanted to the bone defect site to regenerate the defective bone tissue.

The magnetic biomolecule-metal ion self-assembly complex for treatment according to the present embodiment may be a self-assembly complex formed by bonding self-assemblies as monomers to each other, wherein each self-assembly may include iron ion and the ligand that is self-assembled with the iron ion via the first bond. Furthermore, the magnetic biomolecule-metal ion self-assembly complex for the treatment may be formed by self-assembling neighboring self-assemblies with each other via the second bond.

The self-assembly may be self-disassembled into the iron ion and the ligand depending on the surrounding environment of the self-assembly, and furthermore, the rate of the self-disassembly may be controlled.

The ligand may include at least one of AMP (adenosine monophosphate) and ATP (adenosine triphosphate). For example, when the self-assembly is self-disassembled into the iron ion and the ligand, the iron ion or the ligand may be released therefrom. Furthermore, controlling the self-disassembly rate of the self-assembly may allow a rate at which the iron ion or the ligand is released therefrom to be controlled.

A size of the biomolecule-metal ion self-assembly complex may be controlled by controlling the preparation method thereof, that is, the concentration of the material containing the iron ion, the concentration of the material containing the ligand.

The self-assembly has paramagnetic properties, and its movement may be controlled under an externally applied magnetic field thereto. The movement of the biomolecule-metal ion self-assembly complex may be controlled by means outside the body. Further, even when the biomolecule-metal ion self-assembly complex is implanted in the body, the position and the movement speed of the biomolecule-metal ion self-assembly complex may be controlled under the magnetic field applied from an external source thereto.

When the ligand is at least one of AMP and ATP, the self-assembly induces death of cancer cells via the ferroptosis. When the ligand is ATP, the self-assembly may prevent, improve, or treat osteoarthritis. Furthermore, when the ligand is AMP, the self-assembly may be transplanted to the bone defect site to regenerate the defective bone tissue.

Hereinafter, Present Examples and Comparative Examples of the present disclosure are described. However, the following Examples are only preferred embodiments of the present disclosure, and the scope of rights of the present disclosure is not limited to the following Examples.

### Preparing self-assembly

### 1. Materials

Adenosine-5'-monophosphate disodium salt (CAS: 4578-31-8, Alfa Aesar)
Adenosine 5'-triphosphate disodium salt (CAS: 51963-61-2, Daejeong Reagent)
Ferric chloride (Iron(II) chloride, 97 %, Sigma-Aldrich, 25 g, cat. No. 372870)
PMMA (poly(methyl methacrylate), diameter 125 to 150µm, Bangs Laboratories, BB05N)

### 2. Preparing Fe-ATP self-assembly

### Preparing Example 1

To synthesize the Fe-ATP self-assembly, a FeCl₂ solution and an ATP (adenosine triphosphate) solution were prepared using deionized (DI) water. The FeCl₂ solution was prepared by adding 25.35 mg of ferric chloride (Iron(II) chloride, 97%, Sigma-Aldrich, 25g, cat. No. 372870) to 10 mL of deionized water. The ATP solution was prepared by adding 110.23 mg of adenosine 5'-triphosphate disodium salt (CAS: 51963-61-2, Daejeong Reagent) to 10 mL of deionized water.

1 mL of 20mM FeCl₂ solution was added to a reactor containing 1mL of 20mM ATP solution and were mixed with each other in a vortex mixer (Vortex Genie 2, Scientific Industries) to prepare a mixed solution. The prepared mixed solution was maintained at room temperature for 12 hours to induce self-assembly between Fe²⁺ and ATP to prepare the Fe-ATP self-assembly. The solution containing the Fe-ATP self-assembly was centrifuged at 10000 rpm for 5 minutes. To remove unreacted reagents therefrom, a supernatant was discarded, deionized water was added thereto, and the solution was centrifuged. This process was performed twice. After washing, 112.6 mg of the Fe-ATP self-assemblies in the particle form were dispersed in 2 mL of deionized water. The prepared Fe-ATP self-assembly is shown in Table 1.

### Preparing Example 2

A Fe-ATP self-assembly was prepared in the same manner as Preparing Example 1, except that when preparing a mixed solution, 1mL of 1mM FeCl₂ solution was added to a reactor containing 1mL of 1mM ATP solution, and reaction was performed on the prepared mixed solution at room temperature for 30 minutes. The prepared Fe-ATP self-assembly is shown in Table 1.

### Preparing Example 3

A Fe-ATP self-assembly was prepared in the same manner as Preparing Example 1, except that when preparing a mixed solution, 1mL of 0.1mM FeCl₂ solution was added to a reactor containing 1mL of 0.1mM ATP solution, and reaction was performed on the prepared mixed solution at room temperature for 30 minutes. The prepared Fe-ATP self-assembly is shown in Table 1.

**Table 1**

| Example | ATP solution concentration | FeCl₂ solution concentration | Average diameter |
|---|---|---|---|
| Preparing Example 1 | 20mM | 20mM | 600nm |
| Preparing Example 2 | 1mM | 1mM | 150nm |
| Preparing Example 3 | 0.1mM | 0.1mM | 70nm |

### Preparing Example 4

To synthesize the Fe-AMP self-assembly, a FeCl₂ solution and an AMP (adenosine monophosphate) solution were prepared using deionized (DI) water. The FeCl₂ solution was prepared by adding 25.35 mg of ferric chloride (Iron(II) chloride, 97%, Sigma-Aldrich, 25g, cat. No. 372870) to 10 mL of deionized water. The AMP solution was prepared by adding 78.24 mg of AMP (adenosine monophosphate) to 10 mL of deionized water.

1 mL of 20mM FeCl₂ solution was added to a reactor containing 1mL of 20mM AMP solution and were mixed with each other in a vortex mixer (Vortex Genie 2, Scientific Industries) to prepare a mixed solution. The prepared mixed solution was maintained at room temperature for 12 hours to induce self-assembly between Fe²⁺ and AMP to prepare the Fe-AMP self-assembly. The solution containing the Fe-AMP self-assembly was centrifuged at 10000 rpm for 5 minutes. To remove unreacted reagents therefrom, a supernatant was discarded, deionized water was added thereto, and the solution was centrifuged. This process was performed twice. After washing, 89.24 g of the Fe-AMP self-assemblies in the particle form were dispersed in 2 mL of deionized water.

### 4. Fe₃O₄ synthesis

### Preparing Example 5

5.4 g of iron (III) chloride hexahydrate (FeCl₃·6H₂O) and 18.3 g of sodium oleate were added to a mixed solvent composed of 30 mL of deionized water (DI), 40 mL of ethanol (EtOH), and 70 mL of hexane to prepare a mixture. This mixture was heated at 60°C for 8 hours, and an iron-oleate complex contained in an upper hexane layer was washed with deionized water. The hexane was evaporated away to obtain dried iron-oleate complex. The iron-oleate complex was then mixed with 0.14 g of oleic acid and 5 g of 1-octadecene. This mixed solution was stirred vigorously at 320°C for 30 minutes and then cooled to 25°C. Fe₃O₄ nanoparticles synthesized in the cooled mixed solution were repeatedly washed with ethanol and then centrifuged to prepare Fe₃O₄ nanoparticles with an average diameter of 10 nm.

### Evaluation method of basic performance of self-assembly

### 1. Identification of Fe or ATP release from the Fe-ATP self-assembly

Fe and ATP release were identified using the Fe-ATP self-assembly.

The FeCl₂ solution was prepared by adding 25.35 mg of ferric chloride (Iron(II) chloride, 97%, Sigma-Aldrich, 25g, cat. No. 372870) to 10 mL of deionized water, and the ATP solution was prepared by adding 110.23 mg of adenosine 5'-triphosphate disodium salt (CAS: 51963-61-2, Daejeong Reagent) to 10 mL of deionized water.

Each of 5 mL of 20mM ATP solution and 5 mL of 20mM FeCl₂ solution were added from a falcon tube to a vortex mixer (Vortex Genie 2, Scientific Industries) and were mixed with each other using the mixer for 1 minute to prepare a mixed solution. The prepared mixed solution was maintained at room temperature for 12 hours. Then, the mixed solution was centrifuged at 10000rpm for 5 minutes. A supernatant containing unreacted reagents was removed therefrom. 10 mL of deionized water was added thereto. After the adding of the deionized water, centrifugation was performed thereon again at 10,000 rpm for 5 minutes, and a supernatant was removed therefrom, and the Fe-ATP self-assembly as a solid material was collected. The removed supernatant was used to measure ATP loading efficiency based on a value obtained by subtracting an amount of the unloaded ATP from an amount of the initially added ATP via HPLC. 1 mL of PBS solution (pH 7.4 or pH 5.5) was added to the collected Fe-ATP self-assembly and a resulting solution was transferred to a dialysis bag (SnakeSkinTM Dialysis Tubing, 7kDa MWCO).

The dialysis bag containing the Fe-ATP self-assembly was input into a vial containing 4 mL of PBS solution (pH 7.4 or pH 5.5) and was stored at room temperature. From the vial containing the dialysis bag containing the Fe-ATP self-assembly, 40 µL of a supernatant was collected on days 1, 3, 5, and 7, and the concentration of the released Fe or concentration of the released ATP in the collected supernatant was measured and calculated. In this regard, a PBS solution was added to the vial to keep an overall volume constant.

A cumulative ATP release profile was obtained using HPLC (Arc HPLC Core System, Waters) with an analytical column (XBridge BEH C18, 130 Å, 4.6 mm × 250 mm, particle size 5 µm). A mobile phase A and a mobile phase B were prepared as follows. The mobile phase A was obtained by dissolving 0.06 mol/L K₂HPO₄, 0.04 mol/L KH₂PO₄, and 0.1 mol/L KOH in deionized water (pH 7.0). The mobile phase B was a PBS solution.

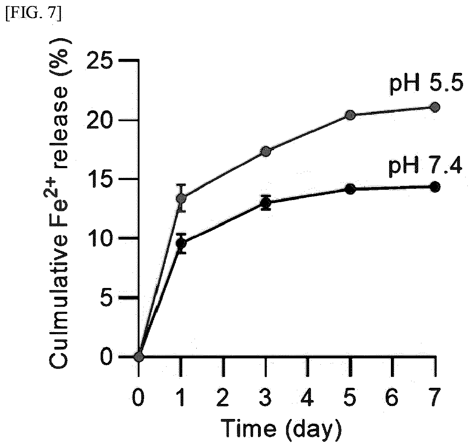

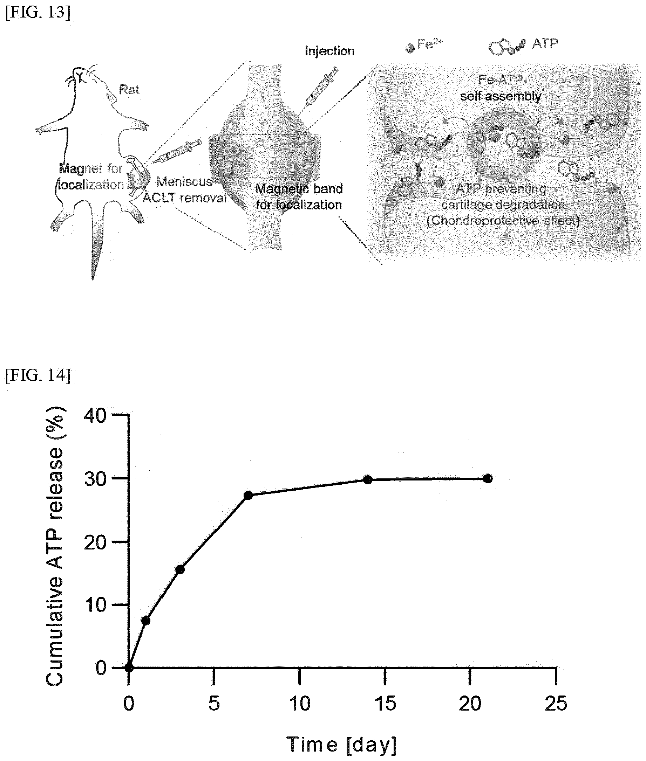

An elution program is as follows. The mobile phase A/the mobile phase B = 100/0(v/v) was performed for 2 minutes; A/B = 95/0 (v/v) was performed for 2 minutes; A/B = 80/20 (v/v) was performed for 2 minutes; A/B = 75/25 (v/v) was performed for 1.3 min; and A/B = 100/0 (v/v) was performed for 1.7 minutes, and then, A/B = 100/0 (v/v) was kept constant again for 1 minute. A isocratic flow rate was 1.2 mL/min, and an injected sample volume was 20 µL, and UV-vis absorbance was monitored at 260 nm. Furthermore, Fe analysis (QuantiChrom^{™} Iron Assay Kit, Bioassay systems) was used to obtain a cumulative Fe release profile at various pHs (FIG. 7, and FIG. 14).

### 2. 1. Identification of AMP release from the Fe-AMP self-assembly

The AMP release were identified using the Fe-AMP self-assembly.

The cumulative AMP release profile was acquired, and sustained AMP release for AMP-induced fracture healing we identified based on the acquired profile. To prepare the Fe-AMP self-assembly, instead of using adenosine 5'-triphosphate disodium salt (CAS: 51963-61-2, Daejeong Reagent), adenosine-5'-monophosphate disodium salt (CAS: 4578-31-8, Alfa Aesar) was used. Further, the Fe-AMP self-assembly was prepared and collected in in the same manner as the manner in which the sample to identify the Fe or ATP release was prepared and collected.

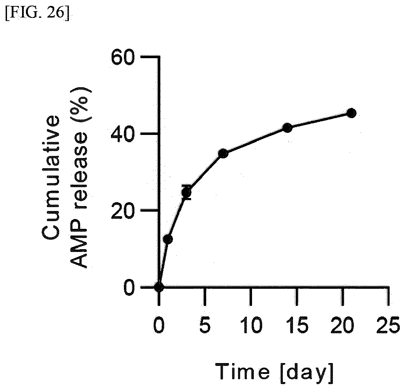

From a vial containing a dialysis bag containing the Fe-AMP self-assembly, 400 µL of a supernatant was collected on days 1, 3, 7, 14, and 21, and the concentration of the released AMP in the collected supernatant was measured via HPLC. The cumulative AMP release profile was obtained by HPLC in the same manner as the manner used in the cumulative ATP release test (see FIG. 26 below).

### 3. IVIS L-012 ROS signal analysis

Luminescent probes L-012 (Wako Chemical, Korea) were dissolved in UPW (Ultra Pure Wate) and a thus prepared solution was maintained in a fresh state just before the experiment. In a 50 *µ*ℓ injection volume, 25 mg/kg thereof was administered subcutaneously to a back of a mouse.

Chemiluminescence (CL) release assessment was started immediately after the injection of L-012 using indiGO^{™} software and IVIS (In vivo Imaging System, NightOWL II LB 983, BERTHOLD Technologies GmbH, Germany). To collect sufficient CL release data, the mouse was exposed thereto for 20 seconds each time. CL released from the mouse skin was recorded and visualized in real time, and then the CL released in an area of interest was quantified as total flux (photons/second).

### Evaluation method of cancer cell death, osteoarthritis and bone defect treatment using self-assembly

### Experimental Example 1 (cancer cell death, osteosarcoma model)

To synthesize the Fe-ATP self-assembly, a FeCl₂ solution and an ATP (adenosine triphosphate) solution were prepared using deionized (DI) water. 5mL of 40mM FeCl₂ solution and 5mL of 40mM ATP (adenosine triphosphate) solution were mixed with each other in deionized water to prepare the mixed salutation at various concentrations. 5mL of 20mM FeCl₂ solution and 5mL of 20mM ATP (adenosine triphosphate) solution at the same concentration were input into a vortex mixer (Vortex Genie 2, Scientific Industries) and were mixed with each other for 1 minute using the mixer to prepare the mixed solution. The mixed solution was maintained at room temperature for 12 hours to induce the self-assembly therebetween to prepare the Fe-ATP self-assembly. The solution containing the Fe-ATP self-assembly was centrifuged at 10000 rpm for 5 minutes. To remove unreacted reagents therefrom, a supernatant was discarded therefrom, deionized water was added thereto, and the solution was centrifuged. This process was performed twice. After washing of a resulting product, 5 mg of the thus collected Fe-ATP self-assembly particles were dispersed in 1 mL deionized water, and, then, 20 µL thereof was injected into the mouse.

The mouse used in this experiment was 8-week-old male Balb/c nude mouse (Orient, Seongnam, South Korea). To identify the efficacy of the Fe-ATP self-assembly in osteosarcoma, PBS (Phosphate-buffered saline), ATP (Daejeong reagent, CAS: 51963-61-2), DOX (doxorubicin), Fe-ATP, Fe-ATP (mag), Fe₃O₄, and Fe₃O₄ (mag) were compared with each other as shown in a following table 2. In this regard, each of 5mg/kg of Fe-ATP and 7mg/kg of Fe₃O₄ was injected thereto.

**Table 2**

| Treatment condition | Details | Particle size | Application of magnetic field |
|---|---|---|---|
| PBS (No treatment) | Welgene, LB 204-02 | - | x |
| DOX | AD mycin, Boryung Pharmaceutical, CAS # 23214-92-8 | - | x |
| Fe-ATP | Synthesized Fe-ATP self-assembly | 600nm | x |
| Fe-ATP (mag) | Synthesized Fe-ATP self-assembly | 600nm | o |
| Fe₃O₄ | Preparing Example 5 | 10nm | x |
| Fe₃O₄ (mag) | Preparing Example 5 | 10nm | o |

Before performing a surgery, the mouse was anesthetized by injecting a mixture of 50 to 70% medical oxygen and 2% isoflurane (CAS # 26675-46-7) thereto at 2 L/min. After making a skin incision in the knee area of the mouse, the knee joint was identified. We exposed the patella tendon and then made an incision thereof to expose the upper portion of the tibia. Afterwards, a hole was made in the exposed site with a needle, and the previously prepared KHOS osteosarcoma cells (1x 107/mL, 100uL) were injected into the bone marrow and the skin was sutured. After injecting the osteosarcoma cells into the mouse, the antibiotic enrofloxacin (Baytril, Bayer, CAS # 93106-60-6) was diluted 5-fold in physiological saline and the injection thereof was administered thereto once a day for 5 days after surgery to prevent infection. The analgesic Ketoprofen (Ketoprofen, Unibio Co. Ltd, CAS # 22071-15-4) was administered thereto for 1 day after surgery to relieve pain.

Before injecting the Fe-ATP self-assembly, a mixed gas of oxygen and 2% isoflurane was injected thereto at 2 L/min to anesthetize the mouse via inhalation. Four weeks after the surgery, the drug was injected thereto 6 times for a total of 6 weeks at 7-day intervals according to the group listed in Table 2. 20 µL of the Fe-ATP self-assembly was injected into each tumor site. In order to compare the effect thereof with that of the existing chemotherapy drug (chemoagent), 2mg/kg of Doxorubicin (Doxorubicin hydrochloride, AD mycin, Boryung Pharmaceutical, CAS # 23214-92-8) was administered thereto. The Fe₃O₄ group was prepared such that 5 mg/kg of Fe ions were injected into the mouse and then was injected thereto in the same volume of 20 µL. In this regard, the injection amount of the Fe-ATP self-assembly was set to 5mg/kg (self-assembly weight/mouse weight), and the injection amount of Fe₃O₄ was set to 7mg/kg (self-assembly weight/mouse weight). As a comparative example, the PBS group was injected thereto at the same volume of 20 µL of PBS (pH 7.4). To identify magnetic targeting, a 270 mT circular neodymium magnet (diameter 5 mm, height 2 mm) was fixed to the injection site with Tegaderm and a surgical tape in each group indicated with (mag).

After 6 weeks, the mouse was euthanized using CO₂, a tumor tissue of a leg site was collected as a sample therefrom, and histology was performed thereon (Department of Pathology, Korea University, Seoul, Korea).

### Experimental Example 2 (osteoarthritis model)

1 mg of the Fe-ATP self-assembly particles prepared in the same manner as in Experimental Example 1 were dispersed in 10 mL of deionized water, and 20 µL thereof was injected into a rat.

The rat used in this experiment was an 8-week-old Sprague-Dawley rat. To identify the efficacy of the Fe-ATP self-assembly in osteoarthritis, PBS (Phosphate-buffered saline) (No treatment), Fe-ATP and Fe-ATP (mag) were compared with each other as shown in a following table 3. In this regard, the injection amount of the Fe-ATP self-assembly was set to 8 *µ*g/kg (self-assembly weight/rat weight). Since the synovial fluid of one rat was approximately 100 µL, 2 µg of the Fe-ATP self-assembly was dissolved in 20 µL of PBS and the prepared solution was injected thereto. Referring to the ATP release profile using the Fe-ATP self-assembly (see FIG. 14), approximately 7% of ATP is released from the Fe-ATP self-assembly for 1 day at room temperature, and approximately 28% of ATP is released therefrom over 7 days. Therefore, when 8 *µ*g/kg (self-assembly weight/rat weight) of the Fe-ATP self-assembly is injected into the rat, the ATP concentration in the synovial fluid of the rat becomes 5 to 8µM, thereby promoting M2 macrophages.

**Table 3**

| Treatment condition | Details | Particle size | Application of magnetic field |
|---|---|---|---|
| PBS(No treatment) | Welgene, LB 204-02 | - | x |
| Fe-ATP | synthesized Fe-ATP self-assembly | 600nm | x |
| Fe-ATP (mag) | synthesized Fe-ATP self-assembly | 600nm | o |

The experiment was performed on the rat in a following order. Before the experiment was performed, the imported animals were subjected to a 7-day quarantine/acclimatization process.

First, we intramuscularly injected 10 mg/kg of alfaxalone (10 mg/kg, CAS # 23930-19-0) and 10 mg/kg of xylazine hydrochloride (10 mg/kg, CAS # 23076-35-9) into the rat. A mixed gas of oxygen and 2% isoflurane was injected thereto at 2 L/min to anesthetize the rat. To prevent infection, the antibiotic enrofloxacin (Baytril, Bayer, CAS # 93106-60-6) was diluted 5-fold in physiological saline and then was injected thereto at 5 mg/kg once a day for 5 days after surgery. To relieve the pain, 5 mg/kg of the analgesic Ketoprofen (Ketopro inj, Unibio Co. Ltd, CAS # 22071-15-4) was injected intradermally thereto for 1 day after surgery.

To perform anterior cruciate ligament transection (ACLT), the skin was incised at the knee site, the knee joint was identified, and the patella tendon was moved aside and the anterior cruciate ligament was exposed. The exposed anterior cruciate ligament was cut from a center thereof to a 1/3 point thereto with ophthalmic scissors, and the Lachman test was performed thereon to identify whether the anterior cruciate ligament was cut. After washing the knee with physiological saline, treatment was performed according to Table 3. The treatment with the group corresponding to Table 3 was performed by injection thereof into the joint fluid within the joint space. Each group was injected thereto 8 times for a total of 8 weeks at 7-day intervals. 20 µL of the Fe-ATP self-assembly and 20 µL of PBS were injected thereto in an equal volume. Regarding the Fe-ATP (mag) group, 20 µL of the Fe-ATP self-assembly was injected thereto in an equal volume. In order to identify magnetic targeting of the Fe-ATP self-assembly, a 270mT circular neodymium magnet (diameter 8mm, height 2mm) was fixed to the joint site with a surgical tape. After performing the treatment with each group, the skin in the knee area was sutured again, and after 8 weeks, plain radiography, micro-computed tomography (Micro-CT), and histological analysis were performed as follows.

To perform plain radiography (weeks 0, 2, 4, 6, and 8), 10 mg/kg of alfaxalone (10 mg/kg, CAS # 23930-19-0) and 10mg/kg of xylazine hydrochloride (10 mg/kg, CAS # 23076-35-9) was injected intramuscularly thereto, followed by general anesthesia. Then, plain radiography was performed. Plain radiography equipment (Cios Alpha, Siemens) was used to identify ACLT immediately after the surgery and whether surrounding fractures occurred. Afterwards, plain radiography was performed at 2-week intervals for 8 weeks to observe the imaging progress which in turn was scored using the Kellgren-Lawrence score.

To identify Micro-CT (8 weeks) and histology (8 weeks), 10 mg/kg of alfaxalone (10 mg/kg, CAS # 23930-19-0) and 10mg/kg of xylazine hydrochloride (10 mg/kg, CAS # 23076-35-9) was injected intramuscularly thereto, followed by general anesthesia. Then, euthanasia was induced with CO₂ gas. A knee cartilage was collected therefrom and fixed in 4% paraformaldehyde solution. A micro CT scan (Genoss, Suwon, Korea) was performed thereon to evaluate the state of cartilage regeneration.

### Experimental Example 3 (bone defect model)

A 3D-shaped Fe-AMP self-assembly was prepared in a form of a scaffold with micro pores. In order to manufacture the 3D-shaped Fe-AMP self-assembly macroporous bone scaffold, a PMMA (poly(methyl methacrylate) leaching method was used. First, 300 mg of PMMA (diameter 125 to 150 µm, Bangs Laboratories, BB05N) was filled into a cylindrical polyethylene mold (diameter 8 mm). 200 µL of a solution in which 2M FeCl₂ was dissolved in deionized water was added to this mold, followed by gently mixing for 5 minutes with a vortex mixer. Next, 200 µL, of a solution in which 2M AMP was dissolved in deionized water was added to the mold, followed by vigorously mixing for 5 minutes using a vortex mixer. The mold containing the mixture was sealed and maintained at 25°C for 1 day. Afterwards, the 3D-shaped Fe-AMP aggregate was separated from the mold and was impregnated with 50 mL of dichloromethane (DCM) for 3 days under a vibration condition (100 rpm) to leach the PMMA. DCM was exchanged every 24 hours. After 3 days, the 3D-shaped Fe-AMP aggregate in which the Fe-AMP self-assemblies were aggregated with each other into a 3D shape was immersed in 50 mL of deionized water for 30 minutes, followed by drying at room temperature. The 3D-shaped Fe-AMP aggregate was cut to a size of 1 cm or 0.8 cm height and was sterilized for 30 minutes using ultraviolet irradiation before being used to treat the bone.

A rabbit used in this experiment was New Zealand White male, 16 to 20 weeks old. In order to identify the efficacy of bone defect treatment of the 3D-shaped Fe-AMP aggregate, no treatment (control), and Ca-phosphate were used as a comparison group, and no treatment (control), Ca-phosphate, Short Fe-AMP, Short Fe-AMP(mag), and Long Fe-AMP as shown in a following table 4 were applied to the rabbit separately. In this regard, Short Fe-AMP refers to a Fe-AMP aggregate of 0.8 cm, and Long Fe-AMP refers to a Fe-AMP aggregate of 1 cm. In order to identify magnetic targeting, a 270 mT circular neodymium magnet (diameter 8mm, height 2mm) was fixed to the injection site with a surgical tape, which is indicated as (mag).

**Table 4**

| Treatment condition | Details | Size | Application of magnetic field |
|---|---|---|---|
| PBS(No treatment) | Welgene, LB 204-02 | - | x |
| Short Fe-AMP | synthesized Fe-AMP self-assembly | 0.8cm | x |
| Short Fe-AMP(mag) | synthesized Fe-AMP self-assembly | 0.8cm | o |
| Long Fe-AMP | synthesized Fe-AMP self-assembly | 1cm | x |
| Ca-phosphate | SN Biologies, NeoBone | 1cm | x |

Experiments were performed on the rabbit in a following order. Before performing the experiments, the imported animals were subjected to a 7-day quarantine/acclimatization process.

In the production of an extensive bone defect model (0-8 weeks), prior to surgery, deep anesthesia (xylazine+alfalaxone+isoflurane) was performed thereon and antibiotics (enrofloxacin) and painkillers (ketoprofen) were administered thereto. We anesthetized the rabbit via intramuscular injection of xylazine (Xylazine 5mg/kg IM) and alfaxalone (alfaxalone 3mg/kg IV). We performed intradermal injection of the antibiotic enrofloxacin (5mg/kg SC) and the analgesic ketoprofen (5mg/kg IM), and injected 2L of a mixed gas of 2% isoflurane and oxygen the rabbit at 2L/min, such that the rabbit was anesthetized via inhalation.

After confirming that cardiac anesthesia was completed, an incision of approximately 7 cm of the rabbit's thigh was made, and the femur was reached via an anterolateral approach. Afterwards, a location where a 1cm bone defect is to be generated was marked on the femoral shaft, and a hole was made using a metal pin at an resection site. Using a surgical electric saw (Colibri II, Depuy Synthes), resection was partially performed only on a portion of a bone resection site that is to be covered with a metal plate. A 6-hole 2.7 locking compression plate (LCP 2.7 straight (6 holes, L 58mm) - Depuy Synthes) was placed at 3 proximal portions and 2 distal portions of the 1.5cm bone defect to fix metal screws thereto. Then, the metal screws were carefully fixed to the 3 proximal portions and the 2 distal portions so as to avoid breaking the rabbit's bones. Entire resection of the bone resection site where the partial resection had been performed was carefully performed using a surgical electric saw (Colibri II, Depuy Synthes). In this regard, rabbit bones were highly brittle and break easily, and thus the resection was done carefully.

The 3D-shaped Fe-AMP aggregate or bone graft material (main ingredient calcium triphosphate, NeoBone, SN Biologics, Suwon, Republic of Korea) was inserted into the bone defect site, and then fascia suturing and skin suturing were performed. In the case of the Short Fe-ATP (mag) group, in order to control magnetic movement for cell recruitment, a 270 mT circular neodymium magnet (diameter 20 mm, height 2 mm) was positioned alternately at the top and bottom of the surgery site three times each day, and was fixed thereto with Tegaderm and the surgical tape. Afterwards, we waited for the formation and maturation of the induced membrane for 8 weeks. The antibiotic enrofloxacin (5mg/kg SC) and the analgesic ketoprofen (ketoprofen 5mg/kg IM) were injected thereto for 3 days after the surgery. To evaluate the experimental results, plain radiography, micro-computed tomography (Micro-CT), and histology (8 weeks) were performed as follows.

Plain radiography (0, 2, 4, 6, and 8 weeks) was performed using radiography equipment (Cios Alpha, Siemens). Before performing the radiography, intramuscular injection of xylazine (5 mg/kg IM) and alfaxalone (3 mg/kg IV) thereto was performed for anesthesia, and then the radiography was performed. Immediately after the first surgery, whether the metal plate was fixed and whether surrounding fractures occurred were identified. Afterwards, the clinical progress was observed for 4 weeks until the induced membrane matured. When there was an abnormality in the progress, the plain radiography was performed 2 weeks after the first surgery to observe the imaging progress. Afterwards, the plain radiography was performed at 2-week intervals for a total of 8 weeks to observe the imaging progress. The observation result was scored based on the RUST score. At 8 weeks after the surgery, whether the bones were integrated with each other was evaluated based on integrations of three or more of four cortical bones on two plain radiographs (anteroposterior and lateral).

Micro-computed tomography (Micro-CT) examination (0 and 8 weeks) was performed at an institution (Genoss, Suwon, Korea). 8 weeks after the surgery, the rabbit was sacrificed (KCl) and the femur was acquired therefrom, and the was fixed in 4% paraformaldehyde solution, then and a micro-CT thereof was taken, and a volume of a reconstructed bone was evaluated using 3D software (provided by Xenos).

Histology (8 weeks) examination was performed after cardiac anesthesia was performed prior to euthanasia. Euthanasia was performed using potassium chloride (dosage: 2 mmol/kg, IV). The cardiac anesthesia was performed by injecting xylazine 5 mg/kg IM and alfaxalone 3 mg/kg IV at a dose of twice of an indicated dose. After the anesthesia, the euthanasia was induced by injection of potassium chloride (dose: 2 mmol/kg, IV) thereto. After the euthanasia was performed, it was identified whether cardiac arrest had clearly occurred. Organ toxicity and examination of a muscle tissue around the bone were performed by H&E (Department of Pathology, Korea University, Seoul, Korea), and bone immunostaining was performed by IHC (Osteocalcin, Genoss, Suwon, Korea).

### Evaluation result of basic performance of self-assembly, and evaluation result of treatment of cancer cell death, osteoarthritis and bone defect using self-assembly

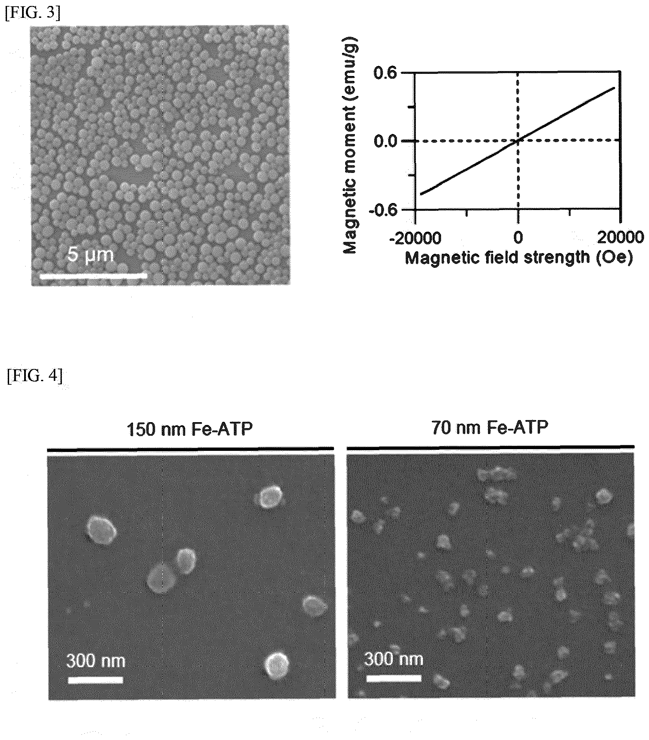

Hereinafter, the evaluation result and the basic performance of the self-assembly prepared according to the example of the present disclosure and the evaluation results of cancer cell killing, osteoarthritis treatment and bone defect treatment using the self-assembly will be described with reference to FIG. 3 to FIG. 32.

FIG. 3 shows the SEM image of the Fe-ATP self-assembly and a result of measuring paramagnetism thereof. The paramagnetic property measurement of the Fe-ATP self-assembly was performed using a vibrating sample magnetometry (VSM). FIG. 3 is an SEM image of the Fe-ATP self-assembly prepared according to Preparing Example 1. It was identified based on FIG. 3 that the Fe-ATP self-assembly was prepared in the form of spherical particles of a uniform size with an average diameter of 600 nm.

It was identified that in the Fe-ATP self-assembly, the iron ion Fe²⁺ and the ATP were self-assembled with each other via the coordination bond to form the self-assembly, and the prepared Fe-ATP self-assemblies were aggregated with each other via the hydrogen bonds or π-π interactions between neighboring Fe-ATP self-assemblies. Furthermore, it was identified that the Fe-ATP self-assembly prepared in an aggregated form had a sphere shape with an almost uniform average diameter. Furthermore, using the magnetic field generated using a 270 mT circular neodymium magnet (diameter 8 mm, height 2 mm), the Fe-ATP self-assembly was moved. In other words, it was identified that the Fe-ATP self-assembly according to Preparing Example 1 had paramagnetism as reversible magnetic characteristic, and was attracted by an external magnet, and thus was displaced.

FIG. 4 is an SEM image of the Fe-ATP self-assembly with an average diameter of each of 150 nm and 70 nm according to one embodiment of the present disclosure. Based on FIG. 4, it was identified that the diameter of the Fe-ATP self-assembly prepared according to each of Preparing Example 2 and Preparing Example 3 was controlled based on the concentration of the iron ion and the concentration of the ATP. Although being prepared in the same way, when the iron ion concentration was set to 1mM and the ATP concentration was set to 1mM as in Preparing Example 2, the average diameter of the Fe-ATP self-assembly was 150nm, whereas when the iron ion concentration was set to 0.1mM and the ATP concentration was set to 0.1mM as in Preparing Example 3, the average diameter of the Fe-ATP self-assembly was 70nm. In other words, when the iron ion concentration was increased or the ATP concentration was increased, the average diameter of the Fe-ATP self-assembly was able to be increased.

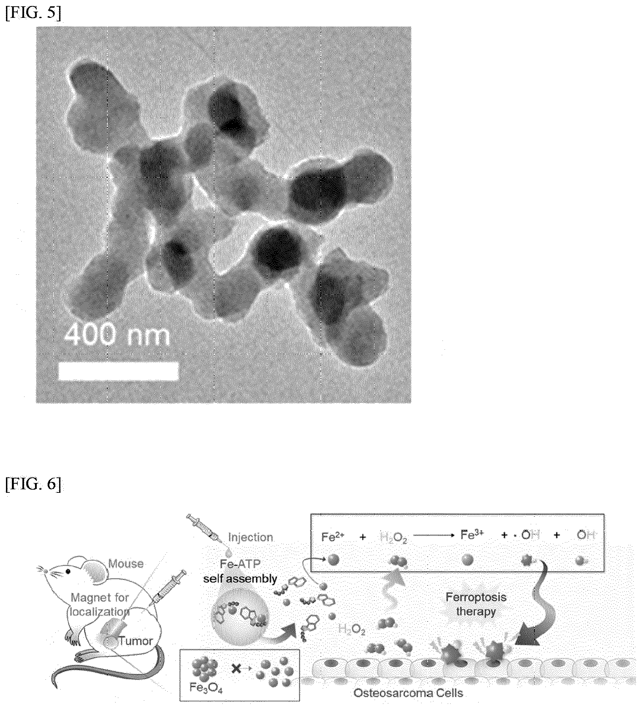

FIG. 5 is a TEM image of the Fe-AMP self-assembly. FIG. 5 shows the Fe-AMP self-assembly prepared using the iron ion and the AMP according to Preparing Example 4. It was identified that the Fe-AMP self-assembly was formed in a form of an 3D-shaped aggregate with micro pores in which the plurality of self-assemblies were aggregated with each other. In other words, it was identified that the Fe-AMP self-assemblies were aggregated with each other and the micro pores were formed between the aggregated Fe-AMP self-assemblies.

Hereinafter, FIG. 6 to FIG. 12 show the experimental results related to Experimental Example 1.

FIG. 6 is a diagram schematically showing the induction of ferroptosis in the cancer cells using the Fe-ATP self-assembly. FIG. 6 is related to the Experimental Example 1, in which the Fe-ATP self-assembly implanted into the body was able to be moved by applying a magnetic force thereto from an external source outside the body. Furthermore, when moving the Fe-ATP self-assembly, it was able to be moved to the targeted cancer cell. Then, the Fe-ATP self-assembly was self-disassembled into the Fe iron and ATP such that the iron ions (Fe²⁺) were continuously released, leading to ferroptosis of the cancer cells. In other words, the Fe-ATP self-assembly according to the present embodiment may be targeted to the specific cancer cells by moving the position of the Fe-ATP self-assembly under the magnetic field, and then may be self-disassembled into the Fe iron and ATP such that the iron ions (Fe²⁺) were continuously released, leading to ferroptosis of the cancer cells. Therefore, the Fe-ATP self-assembly may enable the iron ion-mediated Fenton reaction for cancer treatment using the ferroptosis.

FIG. 7 is a graph showing the iron ion release of the Fe-ATP self-assembly at pH 5.5 or pH 7.4 measured using an iron assay kit. Before identifying the effect of the Fe²⁺ released from the Fe-ATP self-assembly in Experimental Example 1, the Fe²⁺ release from the Fe-ATP self-assembly was identified using PBS which is similar to biological fluid. It was identified that at both pH 5.5 and pH 7.4, the Fe-ATP self-assembly was self-disassembled into the Fe iron and ATP such that the iron ion Fe²⁺ was released therefrom. Furthermore, it was identified that the Fe²⁺ was released in a faster manner and to a greater extent when the pH was 5.5 than when the pH was 7.4. In other words, it is identified that the release of Fe²⁺ is accelerated in a relatively acidic condition, and the release rate and the release amount of Fe²⁺ may be controlled by changing the surrounding condition of the Fe-ATP self-assembly. The release of Fe²⁺ from the Fe-ATP self-assembly according to the present embodiment may be controlled in various ways by designing the Fe-ATP self-assembly in various ways.

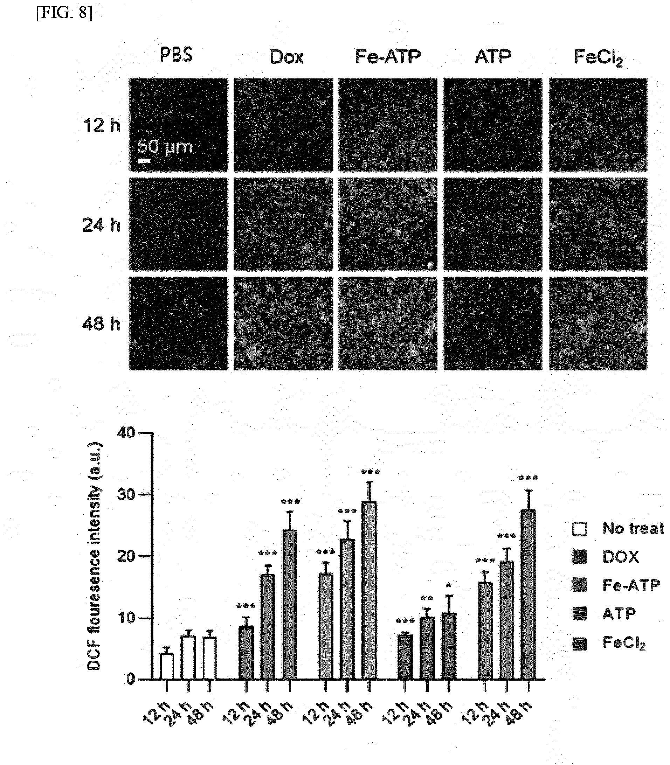

FIG. 8 shows the intracellular ROS concentration measured by DCFDA staining according to each condition. According to Experimental Example 1 below, experiments according to each condition were performed on cells before experiments in mice. As shown in a following table 5, a control group of PBS-only based treatment, a control group of DOX-only based treatment, a control group of ATP-only based treatment, a control group of FeCl₂ based treatment, and the Fe-ATP based treatment were performed on KHOS cancer cells. In this regard, FeCl₂ was disassembled into Fe²⁺ and Cl-. Thus, for the comparison with Fe²⁺ released from the Fe-ATP, the control group of FeCl₂ treatment was added. It was identified that when the Fe-ATP self-assembly was used, ROS generation was promoted compared to the control group (no treatment) in which the same volume of only PBS was injected thereto. Furthermore, when the Fe-ATP self-assembly was used, ROS generation was promoted compared to when ATP alone was used. In other words, this is due to the iron ions released via the self-disassembling of the Fe-ATP self-assembly.

It was identified that the Fe-ATP self-assembly according to the present disclosure generated the ROS more effectively than the toxorubicin (DOX) which is commonly used in cancer cell treatment. FIG. 8 shows cancer cells. In this regard, in the cancer cells, the concentration of hydrogen peroxide therein is higher (10µM-100µM) than that (20nM) in normal cells. In the Fe-ATP self-assembly according to the present embodiment, excessive ROS is generated via the Fenton reaction with the Fe²⁺ released via the self-disassembly, resulting in cancer cell death via the ferroptosis.

**Table 5**

| Treatment condition | Details |
|---|---|
| PBS(No treatment) | Welgene, LB 204-02 |
| DOX | AD mycin, Boryung Pharmaceutical, CAS # 23214-92-8 |
| Fe-ATP | Synthesized Fe-ATP self-assembly(600nm) |
| ATP | Daejeong Reagent, CAS: 51963-61-2 |
| FeCl₂ | Iron (II) chloride, Sigma-Aldrich, cat. No. 372870 |

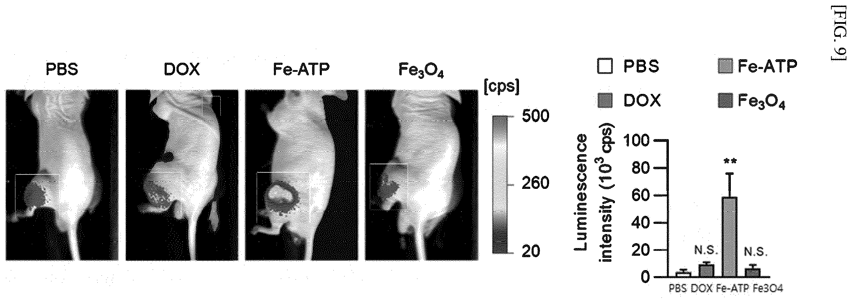

FIG. 9 shows a result of analyzing a IVIS L-012 ROS signal 2 hours after the injection. FIG. 9 shows a result of Experimental Example 1. It was identified based on FIG. 9 that the Fe-ATP self-assembly greatly stimulates ROS production in vivo. PBS as the control group produced almost no ROS, and Fe₃O₄ exhibited similar results to that in the treatment with only PBS. This is because Fe₃O₄ is not disassembled in vivo and does not release Fe²⁺. Furthermore, the Fe-ATP self-assembly according to the present embodiment exhibited higher effectiveness than that of the doxorubicin (DOX). Doxorubicin (DOX) which is used in general cancer treatment has little effect on the ROS release in a short period of time. However, the Fe-ATP self-assembly generates an excessive amount of ROS even in a short period of time, thus making it more effective in killing cancer cells.

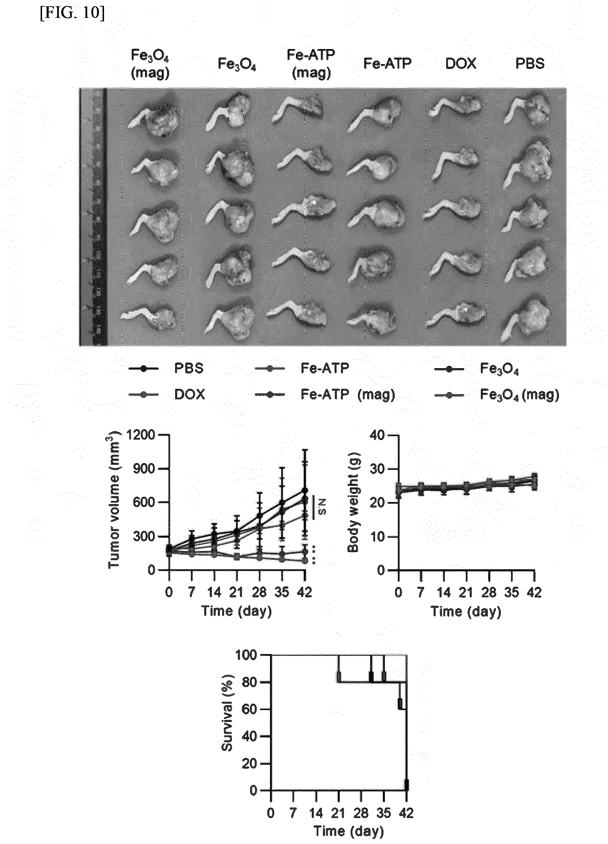

FIG. 10 shows a result of identifying ferroptosis of cancer cells via continuous Fe²⁺ release from the magnetically targeted Fe-ATP self-assembly. It was identified that the Fe-ATP self-assembly exhibited a superior effect in cancer cell treatment than each of PBS and Fe₃O₄ exhibited. Furthermore, it was identified that when an external magnetic field Fe-ATP (mag) was applied to the Fe-ATP self-assembly, the Fe-ATP self-assembly was magnetically targeted, thereby increasing the effectiveness of cancer cell treatment. It was identified that the magnetically targeted Fe-ATP self-assembly inhibited cancer development at a level comparable to that of doxorubicin (DOX) as a commonly used anticancer drug. On the contrary, it was identified that when only Fe₃O₄ was used, this did not inhibit cancer development due to low decomposition efficiency in vivo, regardless of whether it was magnetically targeted.

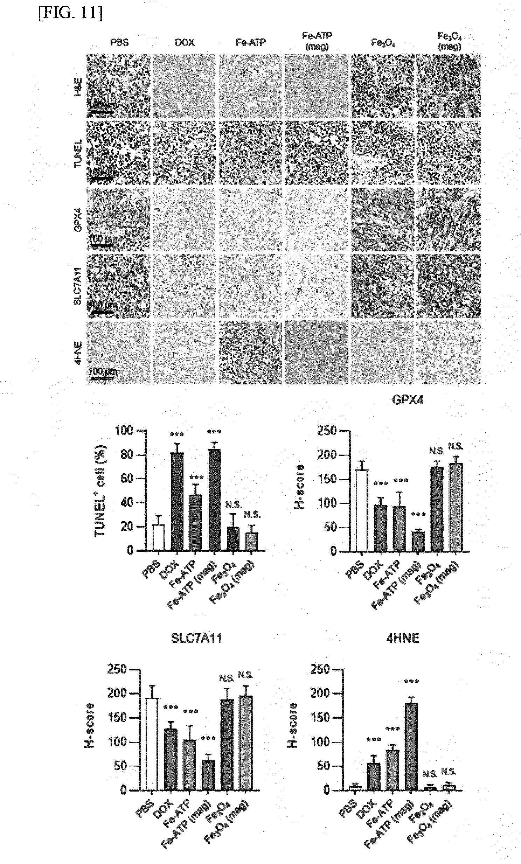

FIG. 11 shows that the magnetically targeted Fe-ATP self-assembly effectively treats osteosarcoma cancer at a level, comparable to that of doxorubicin. In FIG. 11, when many nuclei have a purple color in H&E staining, this means that there are many cancer cells. When many DNA fragments have a brown color in TUNEL staining, many cancer cells have died. Furthermore, a cancer tissue was revealed under H&E staining and TUNEL staining using hematoxylin and eosin. It was identified based on an immunohistochemistry (IHC) staining result that in cancer cell death by the Fe-ATP self-assembly, the released LPO, GPX4, and Xc receptors contribute to the ferroptosis stimulated by the Fenton reaction via Fe²⁺ released from the Fe-ATP self-assembly.

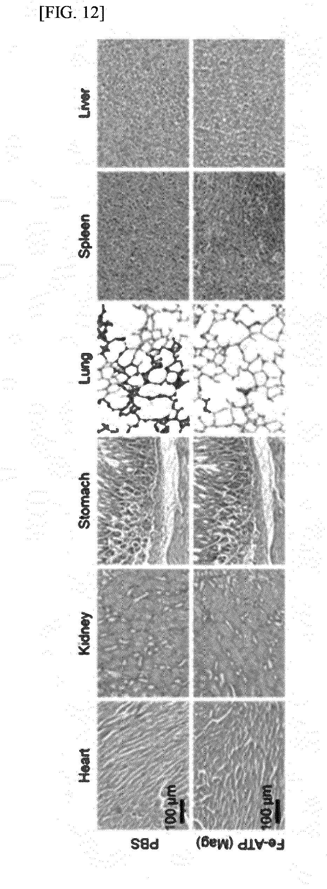

FIG. 12 shows a result of identifying toxicity after transplanting the Fe-ATP self-assembly into the body. In FIG. 12, it was identified that even after implantation of the Fe-ATP self-assembly in an osteosarcoma mouse model under a magnetic field, no toxicity was observed in major organs in vivo. However, the control group exhibited the toxicity at the same level as that of the PBS. In other words, it was identified that when the Fe-ATP self-assembly according to the present embodiment was self-disassembled into the Fe iron and the ATP such that Fe²⁺ was released therefrom, the Fe-ATP self-assembly was not toxic to the human body, and the Fe-ATP self-assembly was not toxic thereto even when the magnetic field was applied thereto.

FIG. 13 to FIG. 22 are directed to experimental results related to Experimental Example 2.

FIG. 13 shows a result of identifying the chondroprotective effect of the magnetically targeted Fe-ATP self-assembly. FIG. 13 shows Experimental Example 2. It was identified based on FIG. 13 that the Fe-ATP self-assembly according to the present embodiment was self-disassembled into the Fe iron and the ATP such that ATP was released therefrom, and the released ATP induced the polarization of anti-inflammatory macrophages and thus was effective in the treatment of osteoarthritis.

FIG. 14 is a graph showing the cumulative ATP release profile from the Fe-ATP self-assembly as measured by HPLC. Before identifying the effect of ATP released from the Fe-ATP self-assembly in Experimental Example 2, the ATP release from the Fe-ATP self-assembly was identified using PBS which was similar to biological fluid. As reviewed above, it was identified that the Fe-ATP self-assembly according to the present embodiment was self-disassembled into the iron ions and ATP such that the iron ion and ATP were released therefrom. Furthermore, it was identified that 30% of ATP was released from the Fe-ATP self-assembly over approximately 25 days. The release of ATP from the Fe-ATP self-assembly according to the present embodiment may be controlled in various ways by designing the Fe-ATP self-assembly in various ways.

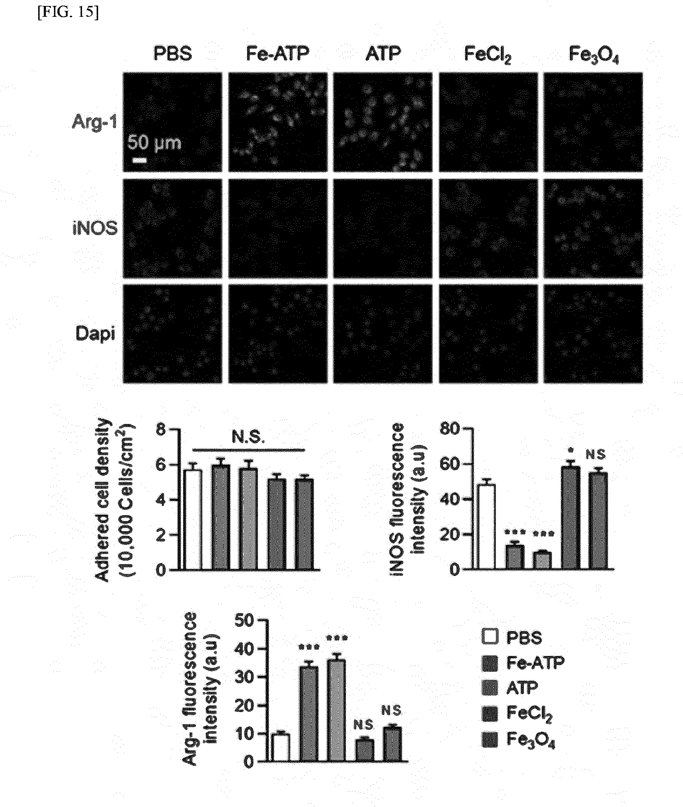

FIG. 15 to FIG. 17 show experiments according to each of the treatment conditions in cells before experimenting in rats according to Experimental Example 2 below. As shown in a following table 6, the conditions were divided into the control group: PBS only based treatment, Fe-ATP based treatment, ATP only based treatment, FeCl₂ based treatment, and Fe₃O₄ based treatment.

**Table 6**

| Treatment condition | Details |
|---|---|
| PBS(No treatment) | Welgene, LB 204-02 |
| Fe-ATP | synthesized Fe-ATP self-assembly(600nm) |
| ATP | Daejeong Reagent, CAS: 51963-61-2 |
| FeCl₂ | Iron(II) chloride, Sigma-Aldrich, cat. No. 372870 |
| Fe₃O₄ | Preparing Example 5(10 nm) |

FIG. 15 is the result of identifying disassembling of the Fe-ATP self-assembly. Referring to FIG. 15, under each treatment condition, an experiment result was identified via immunofluorescence staining of Arg-1 (M2 polarization marker), iNOS (M1 polarization marker), and Dapi. Arg-1 (M2 polarization marker) was not expressed in PBS (no treatment), FeCl₂, and Fe₃O₄, while the M2 polarization marker Arg-1 was strongly expressed only in the Fe-ATP self-assembly and the ATP as samples containing ATP. It was identified that the Fe-ATP self-assembly was disassembled into the ATP and the iron ion such that the ATP was released therefrom, and this enabled ATP-mediated anti-inflammatory polarization of macrophages for osteoarthritis treatment.

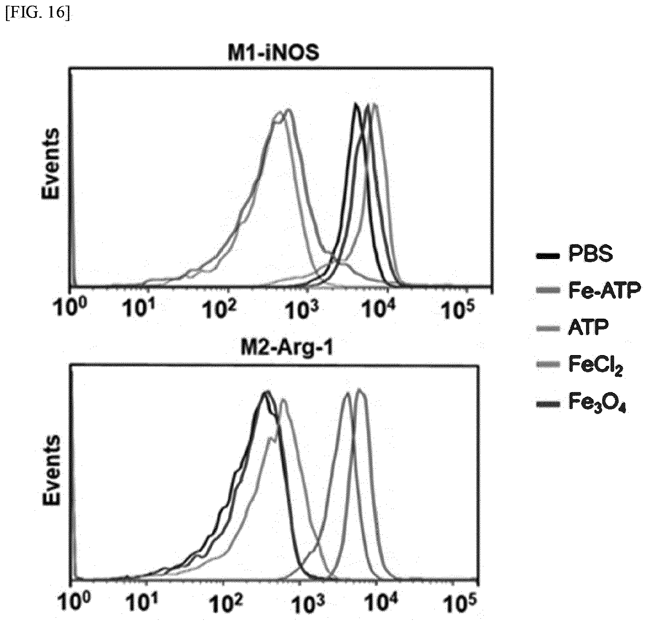

FIG. 16 shows a result of identifying the polarization of macrophages via disassembling of the Fe-ATP self-assembly. Referring to FIG. 16, it was identified that the Fe-ATP self-assembly was disassembled into the ATP and the iron ion such that the ATP was released therefrom, and this enabled ATP-mediated anti-inflammatory polarization of macrophages for osteoarthritis treatment. This was identified through flow cytometry.

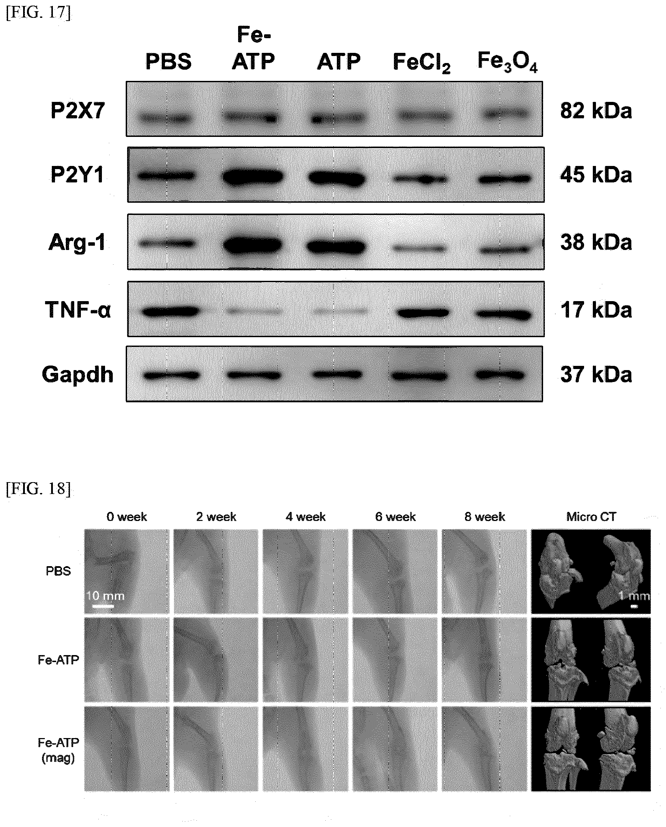

FIG. 17 shows a result of identifying the disassembling of the Fe-ATP self-assembly using western blotting. It was identified that the Fe-ATP self-assembly was disassembled into the ATP and the iron ion such that the ATP was released therefrom, and this enabled AIP-mediated anti-inflammatory polarization of macrophages. It was identified that a low concentration of ATP stimulated P2Y1 signaling which promoted M2 polarization of macrophages, and the low concentration of ATP inhibited P2X7 signaling which inhibited M2 polarization of macrophages.

FIG. 18 shows C-Arm and 3-D micro CT images of the knee joint to identify the effect of the Fe-ATP self-assembly on osteoarthritis treatment. C-Arm images of the knee joint were identified to identify ACLT and a peripheral fracture of an osteoarthritis model at 0, 2, 4, 6, and 8 weeks. 3-D micro-CT images were identified to identify the ACLT and the peripheral fracture in the osteoarthritis model at week 8. The Fe-ATP self-assembly and the magnetically targeted Fe-ATP self-assembly were found to be effective in treating osteoarthritis, compared to the PBS (no treatment).

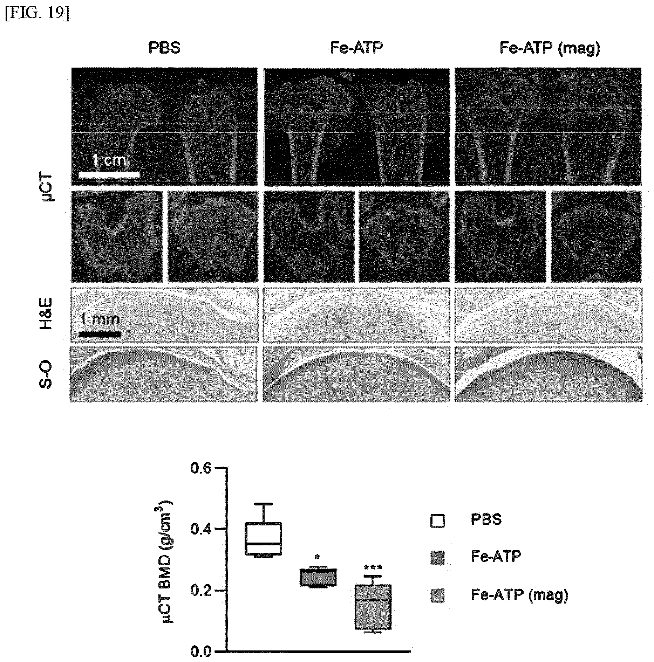

FIG. 19 is a 2-D micro-CT image, and H&E staining, and safranin-o (S-O) staining images of the knee joint of an osteoarthritis model at 8 weeks. Based on FIG. 19, ACLT and peripheral fractures in treating using the Fe-ATP self-assembly according to the present embodiment were identified. It was identified that the treatment using each of the Fe-ATP self-assembly and the magnetically targeted Fe-ATP self-assembly protected the cartilage of the knee joint and prevented the cartilage from being damaged.

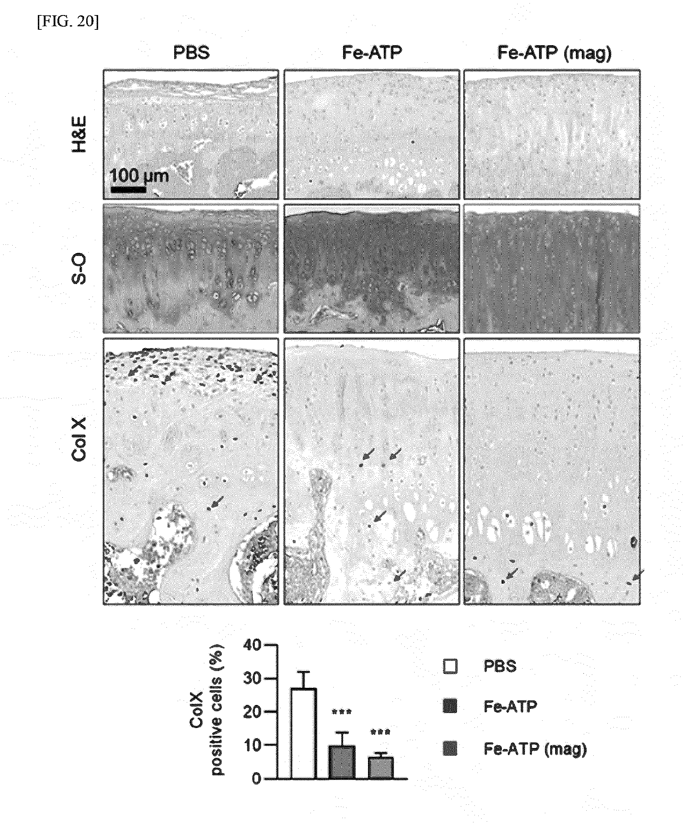

FIG. 20 shows H&E staining, safranin-o (S-O) staining, and collagen X staining images of the knee joint of an osteoarthritis model at 8 weeks. In FIG. 20, ACLT and peripheral fractures were identified in an 8-week-old osteoarthritis model. The Fe-ATP self-assembly and the magnetically targeted Fe-ATP self-assembly alleviated inflammation via anti-inflammatory macrophage polarization and inhibited hypertrophic differentiated chondrocytes that mediate cartilage-to-bone transition. In particular, the magnetically targeted Fe-ATP self-assembly exhibited higher effectiveness than that of the Fe-ATP self-assembly. In this regard, the collagen X stained the hypertrophic differentiated chondrocytes. It was identified that the hypertrophic differentiated chondrocytes promoted cartilage-to-bone transition to worsen the osteoarthritis.

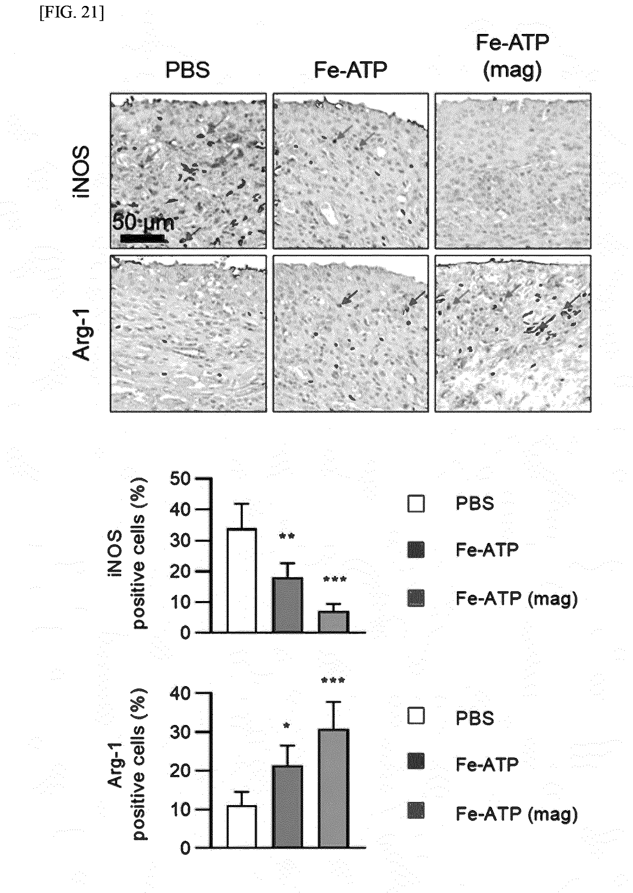

FIG. 21 show iNOS and Arg-1 staining images of the synovial membrane of the knee joint of the osteoarthritis model at 8 weeks. Based on FIG. 21, the ACLT and peripheral fractures in the osteoarthritis model were identified. It was identified that the Fe-ATP self-assembly promoted M2 polarization of macrophages via P2Y1 signaling, thereby protecting the knee joint from cartilage degradation, and in particular, the magnetically targeted Fe-ATP self-assembly was more effective in protecting the knee joint from the cartilage degradation.

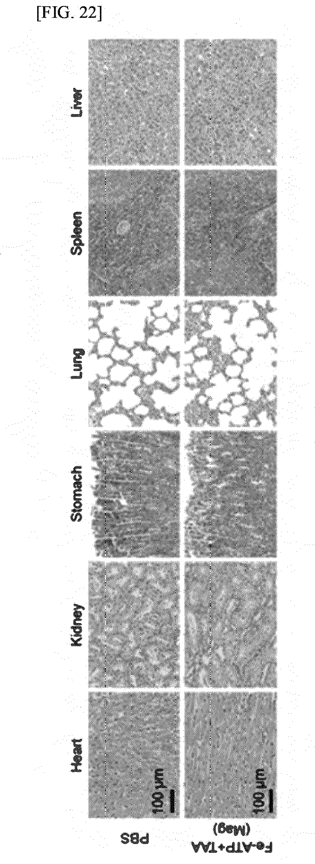

FIG. 22 shows the toxicity result of the Fe-ATP self-assembly as identified in the osteoarthritis rat model (rat model). The toxicity of the Fe-ATP self-assembly was identified after transplantation thereof to various sites under a magnetic field. In this regard, no in vivo toxicity was observed in major organs. In other words, it was identified that the Fe-ATP self-assembly according to the present embodiment was not toxic to the human body even when the Fe-ATP self-assembly was self-disassembled into the ATP and iron ion such that ATP was released therefrom, and, further, the Fe-ATP self-assembly was not toxic thereto even when the magnetic field was applied thereto.

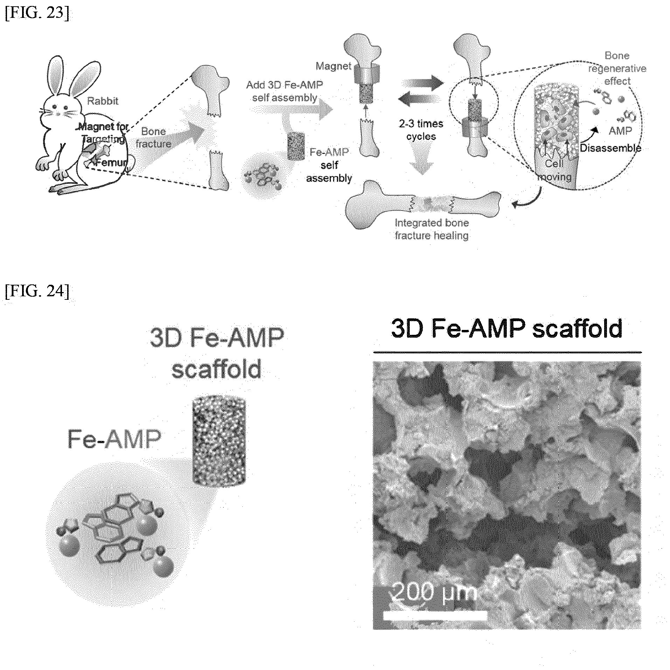

FIG. 23 to FIG. 32 are directed to the experimental results related to Experimental Example 3.

FIG. 23 is a diagram schematically showing the bone regeneration effect of the 3D-shaped Fe-AMP self-assembly.

FIG. 23 relates to Experimental Example 3. Based on FIG. 23, it may be identified that the 3D-shaped Fe-AMP self-assembly exhibits a bone regeneration effect under osteoblast differentiation of stem cells mediated via adenosine-A2B receptor signaling. The 3D-shaped Fe-AMP self-assembly was formed into a three-dimensional macroporous aggregate to enable magnetic movement-based cell recruitment for degraded-AMP-mediated fracture treatment via adenosine-A2B receptor signaling.

FIG. 24 is an SEM image showing a micro pore structure in which the 3D-shaped Fe-AMP self-assembly are aggregated with each other. The Fe-AMP self-assembly according to the present embodiment may be formed in a 3D form with a micro pore structure. The size thereof may be controlled to various sizes from a few micrometers to tens of centimeters. Furthermore, it was identified that he Fe-AMP self-assembly prepared in this way was self-disassembled into the Fe ion and AMP in vitro or in vivo, such that the iron ion Fe²⁺ or AMP was released therefrom.

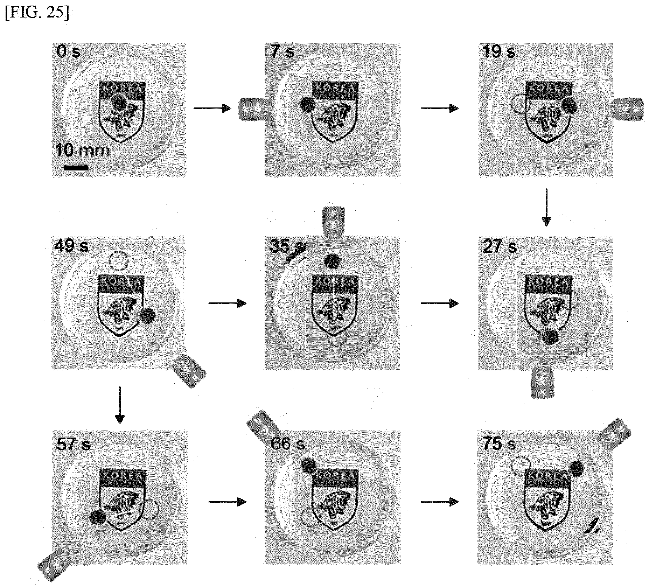

FIG. 25 is a diagram showing magnetic targeting of a 3D-shaped Fe-AMP self-assembly. Referring to FIG. 25, the movement of the 3D-shaped Fe-AMP self-assembly was also able to be controlled when an external magnetic field was applied thereto.

FIG. 26 is a graph showing AMP released from the Fe-AMP self-assembly over time. Before identifying the effect of AMP released from the Fe-AMP self-assembly in Experimental Example 3, the AMP release from the Fe-AMP self-assembly was identified using PBS which is similar to biological fluid. It was identified that the Fe-AMP self-assembly according to the present embodiment was self-disassembled into the Fe ion and the AMM such that the AMP was released therefrom over time. Furthermore, the release lasted for approximately 25 days, and approximately 40% thereof was released therefrom. The release of AMP from the Fe-AMP self-assembly according to the present embodiment may be controlled in various ways by designing the Fe-AMP self-assembly in various ways.

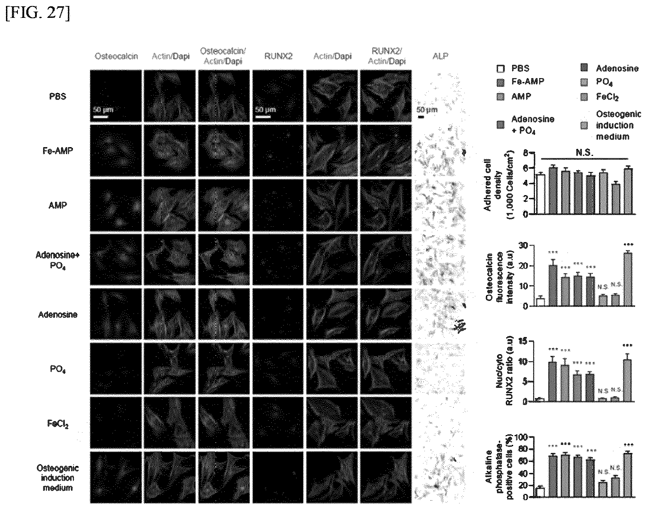

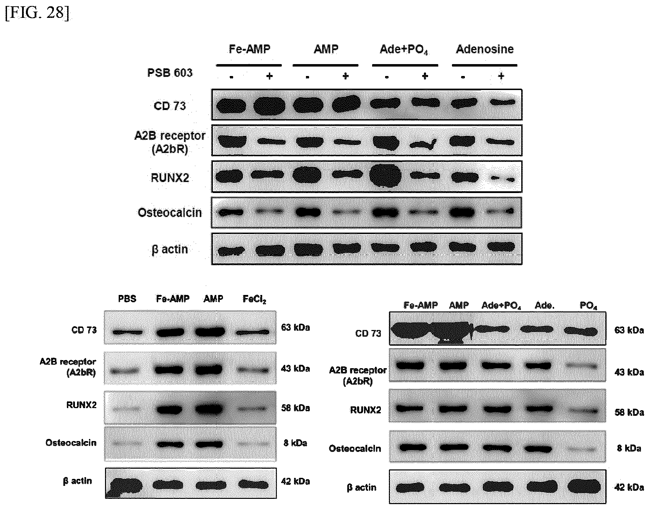

FIG. 27 and FIG. 28 show experiments according to each of treatment conditions in cells before experimenting in rabbits according to Experimental Example 3 below. As shown in a following table 7, the treatment conditions include treatment with only PBS as a control group, treatment with Fe-AMP, treatment with AMP only, treatment with a combination of adenosine and PO₄ (Adenosine+ PO₄), treatment with Adenosine, treatment with PO₄, treatment with FeCl₂, and treatment with an osteogenic induction medium. In this regard, FeCl₂ was disassembled into Fe²⁺ and Cl⁻. Thus, the treatment with FeCl₂ was added to compare the released Fe²⁺ therefrom with the Fe²⁺ released from the Fe-ATP.

**Table 7**

| Treatment agent | Details |
|---|---|
| PBS(No treatment) | Welgene, LB 204-02 |
| Fe-AMP | Synthesized Fe-AMP self-assembly(Preparing Example 4) |
| AMP | Alfa Aesar, CAS: 4578-31-8 |
| Adenosine+PO₄ | Mixture of Daejeong Reagent, CAS: 6613-4405(Adenosine) and Sigma, A9251(PO₄) 1:1 |
| Adenosine | Sigma, A9251 |
| PO₄ | Daejeong Reagent, CAS: 6613-4405 |
| FeCl₂ | Iron (II) chloride, Sigma-Aldrich, cat. No. 372870 |
| Osteogenic induction medium | Mixture of DMEM (Gibco 11965-118), 10mM β-glycerophosphate (sigma G9422), 50µM ascorbic acid-2-phosphate (sigma A8960), and 100 nM dexamethasone (sigma D2915) |

FIG. 27 is the result of identifying that osteogenic differentiation is promoted by adenosine obtained via decomposition of the AMP released from the 3D-shaped Fe-AMP self-assembly. The adenosine obtained via decomposition of the AMP released from the Fe-AMP self-assembly was similar to osteogenic differentiation medium, and was analyzed via actin and dapi-positive nuclei and ALP chemical staining along with immunofluorescence staining of RUNX2 and osteocalcin. The Fe-AMP self-assembly induced osteogenic differentiation of stem cells to a comparable level to that of osteogenic induction medium that promotes the osteogenic differentiation. Adenosine also induced the osteogenic differentiation to a similar level thereto. In other words, the Fe-AMP self-assembly induces osteogenic differentiation of stem cells through adenosine signaling. This was identified via immunofluorescence staining of RUNX2 and osteocalcin along with actin, dapi-positive nuclei, and ALP chemical staining.

FIG. 28 shows the Western blotting results identifying osteogenic differentiation by CD73-mediated adenosine obtained via decomposition of the AMP released from the 3D-shaped Fe-AMP self-assembly. Referring to FIG. 28, it was identified that the CD73-mediated adenosine obtained via decomposition of the AMP released from the Fe-AMP self-assembly promoted osteogenic differentiation via adenosine-A2B receptor signaling, and that adding an A2B receptor inhibitor (PSB 603) allowed adenosine-A2B receptor signaling to be suppressed such that osteogenic differentiation of hMSCs was inhibited, and that CD73 decomposed the AMP into adenosine+PO₄, and the osteogenic differentiation was promoted via signaling of the adenosine-A2B receptor.

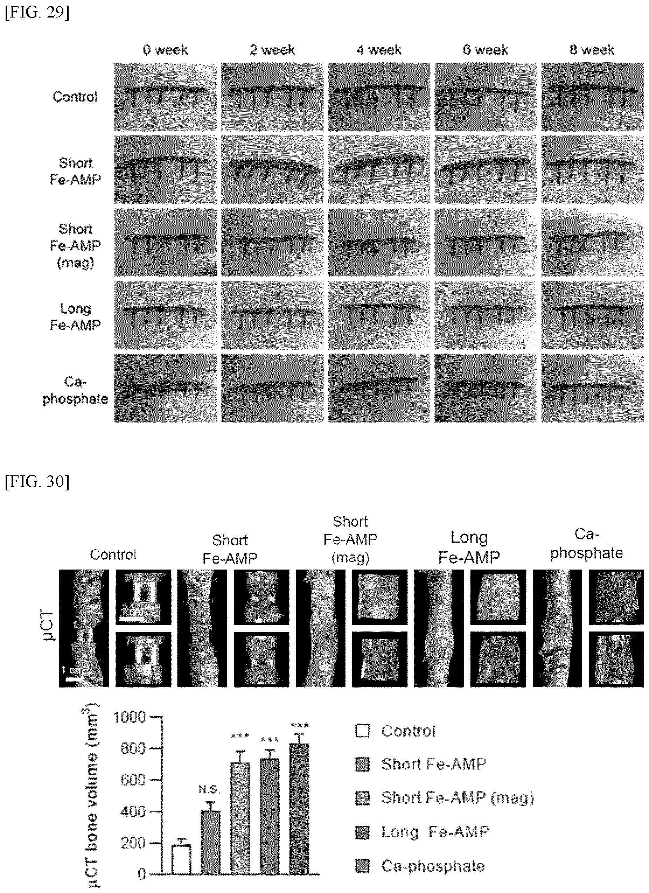

FIG. 29 is a C-Arm image of the femur to identify fractures at 0, 2, 4, 6, and 8 weeks. Referring to FIG. 29, it was identified that the 3D-shaped Fe-AMP self-assembly of the present disclosure exhibited a similar effect to that of Ca-phosphate as a commonly used implant material for fractures as a comparison group. Specifically, Short Fe-AMP self-assembly was effective in treating fractures compared to the control group, while the magnetically targeted Short Fe-AMP(mag) self-assembly was more effective in treating the fractures than the Short Fe-AMP self-assembly was effective. Furthermore, it was identified that each of Short Fe-AMP(mag) self-assembly and Long Fe-AMP self-assembly treated the fractures at a similar effectiveness level to that of Ca-phosphate. In other words, it was identified that when comparing the Short Fe-AMP self-assembly and the Short Fe-AMP(mag) self-assembly with each other, the Short Fe-AMP(mag) self-assembly was moved using a magnet, and thus was more effective in the fracture treatment.

FIG. 30 is a micro-CT image of the femur to identify fractures at 8 weeks. Based on FIG. 30, it was identified that Short Fe-AMP(mag) self-assembly achieved overall fracture treatment due to cell recruitment based on magnetic movement, and reduced AMP-mediated adenosine-A2B receptor signaling. It was identified that Long Fe-AMP self-assembly achieved overall fracture healing, and this was due to decreased AMP-mediated adenosine-A2B receptor signaling. The effectiveness of the fracture treatment was as follows: Long Fe-AMP self-assembly > Short Fe-AMP(mag) self-assembly > Short Fe-AMP self-assembly. Long Fe-AMP self-assembly exhibited similar effectiveness to the effectiveness of the Ca-phosphate. Furthermore, in FIG. 30, the lower the P value, the greater the difference between the two values. Regarding Control vs Short Fe-AMP, p=0.074, regarding Control vs Short Fe-AMP (mg), p<0.001, regarding Control vs Long Fe-AMP, p<0.001, regarding Control vs Ca-phosphate, p<0.001, and regarding Short Fe-AMP vs Short Fe-AMP (mg), P=0.008.

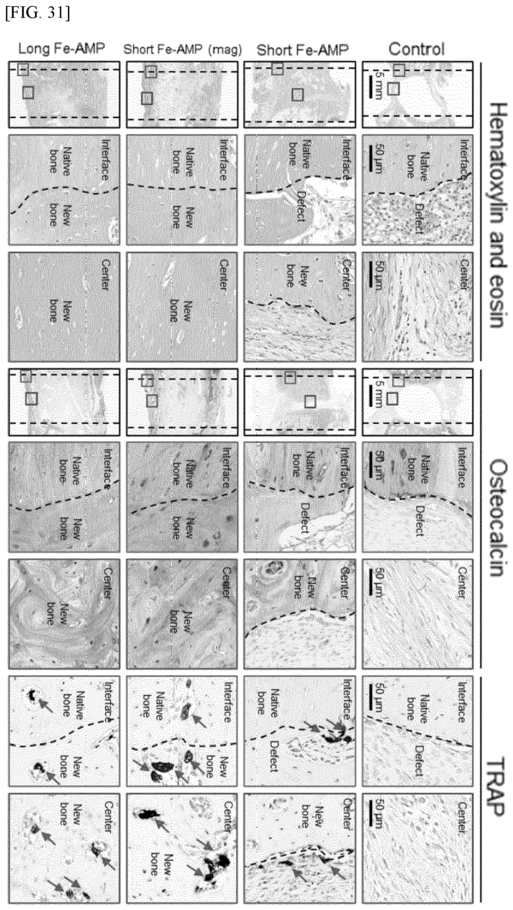

FIG. 31 show H&E, osteocalcin, and TRAP staining images of a fracture. It was identified that The Short Fe-AMP(mag) self-assembly achieved overall fracture treatment due to magnetic movement-based cell recruitment and reduced AMP-mediated adenosine-A2B receptor signaling. It was identified that the Long Fe-AMP self-assembly achieved overall fracture healing, and this was due to decreased AMP-mediated adenosine-A2B receptor signaling.

Referring to FIG. 31, the shape of the tissue was identified based on the H&E staining image, the osteogenic differentiation was identified based on the osteocalcin staining image, and the presence or absence of osteoclasts was identified based on the TRAP staining image. It was identified based on FIG. 31 that in treatment using each of the Long Fe-AMP self-assembly and the Short Fe-AMP(mag) self-assembly, bone was regenerated so as to connect to a middle portion of the bone defect. Considering that the osteoclast is activated, degradation and regeneration of the bone environment are promoted, creating an environment for bone growth. It was identified that because a magnetic field was not applied to the short Fe-AMP self-assembly, the newly generated bone did not fully connect the bone defects to each other.

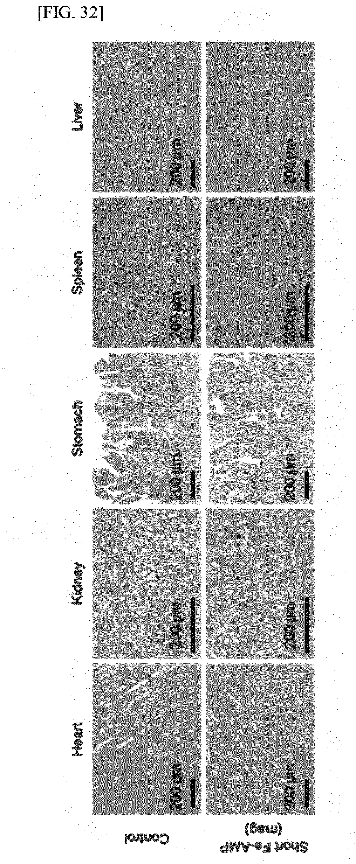

FIG. 32 shows a result of identifying the toxicity of the 3D-shaped Fe-AMP self-assembly in vivo. After implanting the 3D-shaped Fe-AMP self-assembly into a fracture healing rabbit model, the toxicity was identified under a magnetic field. Thus, no toxicity was found in major organs.

As described above, the Fe-ATP self-assembly or the Fe-AMP self-assembly according to the present embodiment may not be toxic in vivo, and may be self-disassembled into the Fe iron and ATP or AMP in vivo or in vitro such that the iron ions and ATP/ADP were released therefrom. The release amount and the release rate thereof may be controlled. Furthermore, the Fe-ATP self-assembly or the Fe-AMP self-assembly according to the present embodiment has paramagnetic properties, and thus may be magnetically targeted by controlling its movement to a specific location under the application of an external magnetic field thereto. Furthermore, even when magnetically targeting the Fe-ATP self-assembly or Fe-AMP self-assembly, no toxicity is found in vivo.

Specifically, the Fe-ATP self-assembly may induce cancer cell death via the ferroptosis and thus may be effective in treating cancer. Furthermore, the Fe-ATP self-assembly may release ATP to treat osteoarthritis. The Fe-AMP self-assembly may act as a graft material of the bone defect site.

A person with ordinary knowledge of the technical field to which the present disclosure belongs will understand that the present disclosure may be implemented in another specific form without changing its technical idea or essential features. Therefore, the embodiments as described above should be understood not as limiting but as illustrative in all respects. The scope of the present disclosure is indicated by the scope of the patent claims as described later rather than the detailed description above, and the meaning and scope of the patent claims and all changes or modified forms derived from the equivalent concept should be interpreted as being included in the scope of the present disclosure.

## Claims

1. A magnetic biomolecule-metal ion self-assembly complex for treatment, the comprising:
an iron ion; and
at least one ligand,
wherein the ligand and the iron ion are reversibly self-assembled with each other or self-disassembled from each other,
wherein the ligand and the iron ion are self-assembled with each other via a first bond to form a self-assembly,
wherein the self-assembling is performed by at least one of the metal ion and the ligand,
wherein the self-assembly includes a plural of self-assemblies,
wherein the self-assemblies adjacent to each other self-bind to each other via a second bond to form the self-assembly complex.

2. The magnetic biomolecule-metal ion self-assembly complex of claim 1, wherein the first bond includes a coordination bond,
wherein the second bond includes at least one of a hydrogen bond and a π-π interaction.

3. The magnetic biomolecule-metal ion self-assembly complex of claim 1, wherein the ligand and the iron ion or the self-assemblies adjacent to each other are self-assembled with each other for a first time duration under a physiologically relevant condition,
wherein the ligand and the iron ion or the self-assemblies adjacent to each other are self-disassembled from each other for a second time duration under a physiologically relevant condition,
wherein the first time duration is in a range of 1 minute to 24 hours,
wherein the second time duration is in a range of 1 day to 90 days.

4. The magnetic biomolecule-metal ion self-assembly complex of claim 1, wherein the ligand includes at least one of phosphate or phosphonate.

5. The magnetic biomolecule-metal ion self-assembly complex of claim 4, wherein the ligand includes at least one of AMP, ADP, ATP, TMP, TDP, TTP, CMP, CDP, CTP, GMP, GDP, GTP, UMP, UDP, UTP, DNA, RNA, AEP (2-aminoethylphosphonic acid), TNA (Throse nucleic acid), GNA (glycol nucleic acid), HNA (1,5-anhydrohexitol nucleic acid), ANA (1,5-anhydroatritol nucleic acid), FANA (2'-deoxy-2'-fluoroarabino nucleic acid) or CeNA (cyclohexenyl nucleic acid).

6. The magnetic biomolecule-metal ion self-assembly complex of claim 4, wherein the ligand includes at least one of AMP (adenosine monophosphate) or ATP (adenosine triphosphate).

7. The magnetic biomolecule-metal ion self-assembly complex of claim 6, wherein when the ligand is ATP (adenosine triphosphate), the magnetic biomolecule-metal ion self-assembly complex is formed in an individual spherical shape,
wherein then the ligand is AMP (adenosine monophosphate), the magnetic biomolecule-metal ion self-assembly complex is formed in a three-dimensional aggregate with micro pores in which the plurality of self-assemblies are aggregated with each other.

8. The magnetic biomolecule-metal ion self-assembly complex of claim 1, wherein the self-assembly has paramagnetic properties,
wherein a movement of the self-assembly is controlled by applying an external magnetic field thereto.

9. The magnetic biomolecule-metal ion self-assembly complex of claim 1, wherein the self-disassembling of the self-assembly is promoted under at least one of a condition containing a chelating agent, a strong acid condition, or a strong base condition,
wherein in the condition containing the chelating agent, the chelating agent is at least one of EDTA (ethylenediaminetetraacetic acid), bipyridyl, or ferrozine,
wherein pH of the strong acid condition is in a range of 2 to 5,
wherein pH of the strong base condition is in a range of 9 to 12.

10. The magnetic biomolecule-metal ion self-assembly complex of claim 1, wherein the self-assembly generates reactive oxygen species (ROS) under a condition containing hydrogen peroxide,
wherein the reactive oxygen species oxidizes cellular phospholipids and inhibits GPX4 (glutathione peroxidase 4) or System xc-cystine/glutamate antiporter (Xc) to induce death of cancer cells via ferroptosis.

11. The magnetic biomolecule-metal ion self-assembly complex of claim 10, wherein the self-assembly has paramagnetic properties, and a movement of the self-assembly is controlled by applying an external magnetic field thereto,
wherein under the application of the external magnetic field thereto, the self-assembly moves to a target cancer cell and induces death of the cancer cell via the ferroptosis.

12. The magnetic biomolecule-metal ion self-assembly complex of claim 10, wherein while the self-assembly induces the death of the cancer cells via the ferroptosis, the self-assembly is not toxic to normal cells.

13. The magnetic biomolecule-metal ion self-assembly complex of claim 1, wherein the self-assembly is used for cancer treatment,
wherein the cancer includes at least one selected from a group consisting of breast cancer, colon cancer, rectal cancer, lung cancer, colon cancer, thyroid cancer, oral cancer, pharynx cancer, larynx cancer, cervical cancer, brain cancer, ovarian cancer, bladder cancer, kidney cancer, liver cancer, pancreatic cancer, prostate cancer, skin cancer, tongue cancer, uterine cancer, stomach cancer, bone cancer, and blood cancer.

14. The magnetic biomolecule-metal ion self-assembly complex of claim 1, wherein the ligand includes ATP (adenosine triphosphate),
wherein the self-assembly are self-disassembled into the ATP and the iron ion such that the ATP is released therefrom to promote M2 polarization of macrophages via a P2Y1 receptor.

15. The magnetic biomolecule-metal ion self-assembly complex of claim 14, wherein the self-assembly promotes M2 polarization of macrophages distributed in a synovial membrane of a bone joint,
wherein the M2 polarization of the macrophages has anti-inflammatory activity of a synovial fluid of the bone joint.

16. The magnetic biomolecule-metal ion self-assembly complex of claim 15, wherein the self-assembly maintains an anti-inflammatory environment of the synovial fluid in the bone joint to protect a bone cartilage to prevent, improve, or treat osteoarthritis.

17. The magnetic biomolecule-metal ion self-assembly complex of claim 15, wherein the self-assembly has paramagnetic properties, and a movement thereof is controlled by applying an external magnetic field thereto,
wherein under the application of the external magnetic field thereto, the self-assembly moves to a target bone joint site to protect the bone cartilage to prevent, improve, or treat osteoarthritis.

18. The magnetic biomolecule-metal ion self-assembly complex of claim 15, wherein the self-assembly promotes M2 polarization of macrophages distributed in the synovial membrane of the bone joint,
wherein the M2 polarization of the macrophage does not exhibit toxicity to normal cells while having the anti-inflammatory activity of the synovial fluid of the bone joint.

19. The magnetic biomolecule-metal ion self-assembly complex of claim 1, wherein the ligand includes AMP (adenosine monophosphate),
wherein the magnetic biomolecule-metal ion self-assembly complex is formed in a three-dimensional aggregate with micro pores in which the plurality of self-assemblies are aggregated with each other,
wherein the three-dimensional aggregate has an average diameter in a range of 500µm to 10cm.

20. The magnetic biomolecule-metal ion self-assembly complex of claim 19, wherein the magnetic biomolecule-metal ion self-assembly in the form of the three-dimensional aggregate is transplanted to a bone defect site and acts as a bone graft material to restore a defective bone tissue.

21. The magnetic biomolecule-metal ion self-assembly complex of claim 19, wherein the self-assembly has paramagnetic properties, and a movement thereof is controlled by applying an external magnetic field thereto,
wherein the self-assembly in the form of the three-dimensional aggregate is transplanted to the bone defect site,
wherein under the application of the external magnetic field thereto, the self-assembly moves in one direction and/or an opposite direction thereto.

22. The magnetic biomolecule-metal ion self-assembly complex of claim 19, wherein the self-assembly is transplanted to the bone defect site,
wherein a host cell attaches to a surface of the self-assembly to form a skeleton.

23. The magnetic biomolecule-metal ion self-assembly complex of claim 22, wherein the self-assembly has paramagnetic properties, and a movement thereof is controlled by applying an external magnetic field thereto,
wherein under the application of the external magnetic field thereto, the host cell attached to the surface of the self-assembly moves together therewith.

24. The magnetic biomolecule-metal ion self-assembly complex of claim 19, wherein the self-assembly is transplanted to a bone defect site,
wherein the self-assembly is self-disassembled into the AMP and the iron ion such that the AMP is released therefrom for 1 to 70 days.

25. The magnetic biomolecule-metal ion self-assembly complex of claim 24, wherein the AMP released from the self-assembly decomposes into adenosine on a surface of the host cell to promote signaling of an adenosine receptor on a surface of the host cell,
wherein the adenosine receptor includes adenosine A2B receptor.

26. The magnetic biomolecule-metal ion self-assembly complex of claim 24, wherein the AMP released from the self-assembly promotes osteogenic differentiation of mesenchymal stem cells (MSCs) via signaling of an adenosine receptor of the mesenchymal stem cells (MSCs),
wherein the osteogenic differentiation of the mesenchymal stem cells (MSC) promotes bone generation.

27. The magnetic biomolecule-metal ion self-assembly complex of claim 20, wherein the self-assembly is not toxic to normal cells while being transplanted to the bone defect site to regenerate the defective bone tissue.

28. The magnetic biomolecule-metal ion self-assembly complex of claim 1, wherein the ligand includes at least one of AMP (adenosine monophosphate) or ATP (adenosine triphosphate),
wherein the self-assembly has paramagnetic properties, and a movement thereof is controlled under an externally applied magnetic field thereto,
wherein when the ligand is at least one of AMP or ATP, the self-assembly induces death of cancer cells via ferroptosis,
wherein when the ligand is ATP, the self-assembly prevents, improves, or treats osteoarthritis,
wherein when the ligand is AMP, the self-assembly is transplanted to a bone defect site to regenerate a defective bone tissue.
